# EUROPEAN PATENT APPLICATION

(11) **EP 4 091 670 A1**
(43) Date of publication of application: **23.11.2022**
(21) Application number: 21767151.0
(22) Date of filing: 10.03.2021
(51) Int. Cl.: A61P 1/04, C07D 471/04, A61K 31/4188

(54) **CRYSTAL OF IMIDAZOPYRIDINONE COMPOUND OR SALT THEREOF**

(30) Priority: 11.03.2020 JP 2020041453
(71) Applicant: KISSEI PHARMACEUTICAL CO., LTD., Matsumoto-shi Nagano 399-8710 (JP)
(72) Inventor: TAKEUCHI, Hideki, Azumino-shi, Nagano 399-8304 (JP); KIJIMA, Yoshiro, Azumino-shi, Nagano 399-8304 (JP); MORIYAMA, Akihiro, Azumino-shi, Nagano 399-8304 (JP)
(74) Representative: Isarpatent
(86) International application number: PCT/JP2021/009405
(87) International publication number: WO 2021/182490

(57) **Abstract**

As a drug substance, a crystal having good physical properties is preferable. However, the crystal form that is most excellent as a drug substance may vary with the compound. In general, it is difficult to predict a crystal form of a drug substance having good physical properties, and it is required to variously examine each compound. Therefore, an object of the present invention is to provide a crystal having good physical properties as a drug substance for a novel compound or a salt thereof.

The present invention relates to a crystal of the compound represented by the following formula (I) or a salt thereof useful for the treatment of an inflammatory bowel disease.

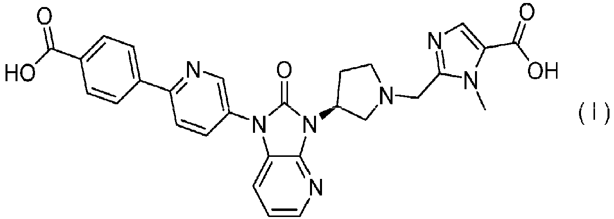

## Description

### [Technical Field]

The present invention relates to a crystal of an imidazopyridinone compound or a salt thereof useful as a medicament.

More particularly, the present invention relates to a crystal of an imidazopyridinone compound or a salt thereof which has a prolyl hydroxylase inhibitory effect and which is useful as an agent for the treatment of an inflammatory bowel disease such as ulcerative colitis.

### [Background Art]

Inflammatory bowel diseases (IBDs) are chronic diseases in which inflammation and ulcers are caused in the intestinal mucosa due to excessive immune response. IBDs include, for example, ulcerative colitis and Crohn's disease.

Ulcerative colitis is a large intestine disease causing diffuse non-specific inflammation of uncertain cause. Large intestine mucosa is ulcerated, and erosion or ulcers may be caused in mucosa. Ulcerative colitis may be divided into "active phase" in which bloody stool, erosion, ulcers and the like are observed and "remission phase" in which the observations of the active phase disappear. Long-term treatment is required because relapse and remission are often repeated in the course.

For the treatment of ulcerative colitis, a 5-aminosalicylic acid formulation (5-ASA) is first used as a standard agent. However, it has been reported that the effectiveness of 5-ASA is approximately 46 to 64% and that patients with remission by administration of 5-ASA are no more than 29 to 45%. When the effect of 5-ASA is not observed, a steroid is used. Immunosuppressive agents, TNF-α antibodies and the like are sometimes used for the treatment of ulcerative colitis in addition to those medicaments. However, all the medicaments have problems such as side effects and necessity for careful administration. Therefore, a therapeutic agent having a novel mode of action for ulcerative colitis is desired.

It has been known that expression of genes associated with barrier function of gastrointestinal epithelium is induced by hypoxia-inducible factor 1α (HIF-1α) in a pathological condition of IBD. HIF-1α is one of the subtypes of hypoxia-inducible factor α (HIF-α). HIF-α is stabilized in a hypoxic environment (Hypoxia), and then it activates the transcription of various genes in response to hypoxia. In contrast, the proline residues of HIF-α are hydrolyzed by prolyl hydroxylases (PHDs) in an oxygen-rich environment (Normoxia), and then the HIF-α is degraded via the proteasomal pathway.

Three subtypes are known for PHDs, namely PHD1, PHD2 and PHD3. AKB-4924 is known as a PHD inhibitor. It has been reported that AKB-4924 has a PHD2 inhibitory effect and stabilizes HIF-1α in large intestine tissues (Non-patent literature 1). Furthermore, AKB-4924 has an improvement effect in TNBS induced colitis model.

In contrast, PHD inhibitors, such as Roxadustat and Daprodustat, have a hematopoietic effect and have been developed as therapeutic agents for anemia (Non-patent literature 2). Thus, it is important to avoid systemic effects such as a hematopoietic effect when a PHD inhibitor is used as a therapeutic agent for IBD.

For example, spiro compounds are described in Patent literatures 1 and 5 and Non-patent literatures 3 and 4 as PHD inhibitors. Compounds including imidazopyridinone are described or illustrated in Patent literatures 2 to 4. However, the crystals of the imidazopyridinone compound or a salt thereof of the present invention are neither described nor suggested in the above literatures.

### [Citation List]

### [Patent Literature]

Patent literature 1: U.S. Published Application No. 2011/ 0152304
Patent literature 2: WO 2009/029609
Patent literature 3: WO 2003/037890
Patent literature 4: WO 2017/066014
Patent literature 5: U.S. Published Application No. 2010/ 0137297

### [Non-patent literature]

Non-patent literature 1: Ellen Marks et al., "Inflamm. Bowel. Dis.", 2015, Vol. 21, No. 2, pp. 267-275
Non-patent literature 2: Mun Chiang Chan et al., "Molecular Aspects of Medicine", 2016, Vol. 47-48, pp. 54-75
Non-patent literature 3: Guanghui Deng et al., "Bioorganic & Medicinal Chemistry", 2013, Vol. 21, pp. 6349-6358
Non-patent literature 4: Petr Vachal et al., "Journal of Medicinal Chemistry", 2012, Vol. 55, pp. 2945-2959

### [Summary of the Invention]

### [Problems to Be Solved by the Invention]

The present applicant developed the compound represented by the following formula (I) (hereinafter, referred to as "compound 1") as a novel compound which has a PHD2 inhibitory effect, and they filed a patent application for the invention (PCT/JP2019/035792). 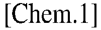 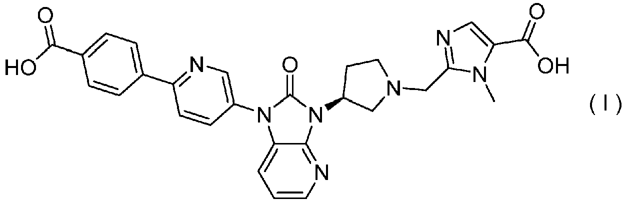 (Chemical name: 2-{[(3S)-3-{1-[6-(4-Carboxyphenyl)pyridin-3-yl]-2-oxo-2,3-dihydro-1H-imidazo[4,5-b]pyridin-3-yl}pyrrolidin-1-yl]methyl}-1-methyl-1H-imidazole-5-carboxylic acid)

As a drug substance, a crystal having good physical properties is preferable. However, the crystal form that is most excellent as a drug substance may vary with the compound. In general, it is difficult to predict a crystal form of a drug substance having good physical properties, and it is required to variously examine each compound. Therefore, an object of the present invention is to provide a crystal having good physical properties as a drug substance for the compound 1, which is a novel compound, or a salt thereof.

### [Means for Solving the Problems]

The present invention relates to a crystal of the compound 1 or a salt thereof which has a PHD2 inhibitory effect and which is useful for the treatment of an inflammatory bowel disease. That is, the present invention relates to the following [1] to [12] and the like.
[1] A crystal of the compound represented by the following formula (I): 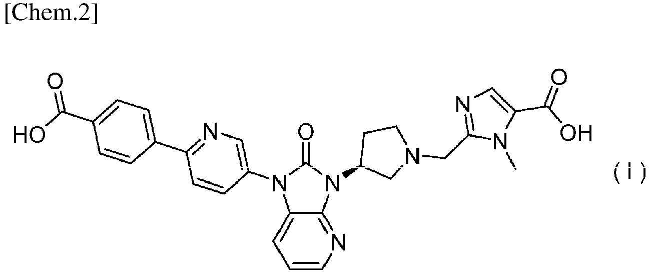 or a crystal of the salt of the compound represented by the formula (I) with an acid or a base selected from the group consisting of methanesulfonic acid, hydrogen chloride, p-toluenesulfonic acid, sodium, potassium and calcium.
[2] The crystal according to the above [1] which is a crystal of the methanesulfonic acid salt having a subset of the peaks at diffraction angles (2*θ* (°)) selected from the group consisting of the following (i) to (iv) in a powder X-ray diffraction:
   (i) peaks of 10.6±0.2 and 14.1±0.2 (crystal form I);
   (ii) peaks of 21.8±0.2 and 25.5±0.2 (crystal form II);
   (iii) peaks of 12.8±0.2 and 22.6±0.2 (crystal form III); and
   (iv) peaks of 6.0±0.2 and 15.4±0.2 (crystal form IV).
[3] The crystal according to the above [1] which is a crystal of the methanesulfonic acid salt having a subset of the peaks at diffraction angles (2*θ* (°)) selected from the group consisting of the following (i) to (iv) in a powder X-ray diffraction:
   (i) peaks of 7.0±0.2 and 10.6±0.2 (crystal form I);
   (ii) peaks of 18.4±0.2 and 25.5±0.2 (crystal form II);
   (iii) peaks of 17.8±0.2 and 22.6±0.2 (crystal form III); and
   (iv) peaks of 6.0±0.2 and 11.0±0.2 (crystal form IV).
[4] The crystal according to the above [1] which is a crystal of the hydrochloride having a subset of the peaks at diffraction angles (2*θ* (°)) selected from the group consisting of the following (i) to (iv) in a powder X-ray diffraction:
   (i) peaks of 13.1±0.2 and 17.4±0.2 (crystal form I);
   (ii) peaks of 6.2±0.2 and 8.1±0.2 (crystal form II);
   (iii) peaks of 12.9±0.2 and 21.4±0.2 (crystal form III); and
   (iv) 10.5±0.2 and 11.0±0.2 (crystal form IV).
[5] The crystal according to the above [1] which is a crystal of a salt having a subset of the peaks at diffraction angles (2*θ* (°)) selected from the group consisting of the following (i) to (iv) in a powder X-ray diffraction:
   (i) a p-toluenesulfonic acid salt, having peaks of 6.1±0.2 and 24.7±0.2;
   (ii) a sodium salt, having peaks of 5.8±0.2 and 22.7±0.2;
   (iii) a potassium salt, having peaks of 5.7±0.2 and 22.8±0.2; and
   (iv) a calcium salt, having peaks of 7.3±0.2 and 16.1±0.2.
[6] The crystal according to the above [1] which is a crystal of the compound having a subset of the peaks at diffraction angles (2*θ* (°)) selected from the group consisting of the following (i) to (iii) in a powder X-ray diffraction:
   (i) peaks of 10.1±0.2 and 18.0±0.2 (crystal form I);
   (ii) peaks of 12.5±0.2 and 17.0±0.2 (crystal form II); and
   (iii) peaks of 11.1±0.2 and 14.1±0.2 (crystal form III).
[7] The crystal according to the above [1] which is a crystal of the compound having a subset of the peaks at diffraction angles (2*θ* (°)) selected from the group consisting of the following (i) to (iii) in a powder X-ray diffraction:
   (i) peaks of 7.6±0.2 and 18.0±0.2 (crystal form I);
   (ii) peaks of 11.4±0.2 and 17.0±0.2 (crystal form II); and
   (iii) peaks of 10.5±0.2 and 11.1±0.2 (crystal form III).
[8] The crystal according to the above [1] which is a crystal of the compound having peaks at diffraction angles (2*θ* (°)) of 7.6±0.2, 9.0±0.2, 10.1±0.2, 13.0±0.2, 17.1±0.2, 18.0±0.2, 19.1±0.2, 21.1±0.2, 21.7±0.2, 23.4±0.2, 26.2±0.2 and 27.5±0.2 in a powder X-ray diffraction (crystal form I).
[9] A pharmaceutical composition comprising the crystal according to any one of the above [1] to [8] and a pharmaceutical additive.
[10] The pharmaceutical composition according to the above [9] which is a pharmaceutical composition for use in the treatment of an inflammatory bowel disease.
[11] The pharmaceutical composition according to the above [10], wherein the inflammatory bowel disease is ulcerative colitis or Crohn's disease.
[12] A crystal of the compound represented by the following formula (I): 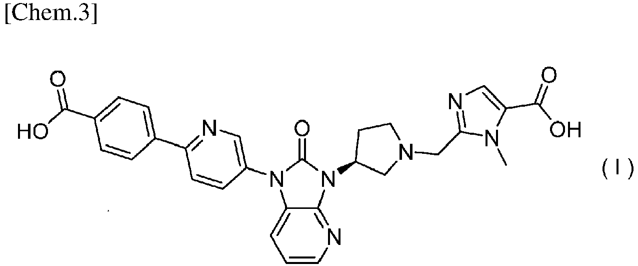 or a salt thereof.

In an embodiment, the present invention relates to a method for treating an inflammatory bowel disease, comprising administering a necessary amount of the pharmaceutical composition according to the above [9] to a patient.

In an embodiment, the present invention relates to a use of the crystal according to any one of the above [1] to [8] for manufacturing a pharmaceutical composition for use in the treatment of an inflammatory bowel disease.

In an embodiment, the present invention relates to the crystal according to the above [1] which is a crystal of the methanesulfonic acid salt having a subset of the peaks at diffraction angles (2*θ* (°)) selected from the group consisting of the following (i) to (iv) in a powder X-ray diffraction:
(i) peaks of 7.0±0.2, 10.6±0.2, 14.1±0.2 and 16.2±0.2 (crystal form I);
(ii) peaks of 18.4±0.2, 18.7±0.2, 21.8±0.2 and 25.5±0.2 (crystal form II);
(iii) peaks of 12.8±0.2, 17.8±0.2, 18.3±0.2 and 22.6±0.2 (crystal form III); and
(iv) peaks of 6.0±0.2, 11.0±0.2, 15.4±0.2 and 16.3±0.2 (crystal form IV).

In an embodiment, the present invention is the crystal according to the above [1] which is a crystal of the compound having a subset of the peaks at diffraction angles (2*θ* (°)) selected from the group consisting of the following (i) to (iii) in a powder X-ray diffraction:
(i) peaks of 7.6±0.2, 10.1±0.2, 13.0±0.2 and 18.0±0.2 (crystal form I);
(ii) peaks of 11.4±0.2, 12.2±0.2, 12.5±0.2 and 17.0±0.2 (crystal form II); and
(iii) peaks of 10.5±0.2, 11.1±0.2, 14.1±0.2 and 24.7±0.2 (crystal form III).

In an embodiment, the present invention relates to the crystal according to the above [1] which is a crystal of the methanesulfonic acid salt having a subset of the peaks at diffraction angles (2*θ* (°)) in a powder X-ray diffraction and endotherm, wherein the subset of the peaks and the endotherm are selected from the group consisting of the following (i) to (iv):
(i) peaks of 7.0±0.2, 10.6±0.2, 14.1±0.2 and 16.2±0.2 and the endothermic peak top around 253°C (crystal form I);
(ii) peaks of 18.4±0.2, 18.7±0.2, 21.8±0.2 and 25.5±0.2 and the endothermic peak top around 248°C (crystal form II);
(iii) peaks of 12.8±0.2, 17.8±0.2, 18.3±0.2 and 22.6±0.2 and the endothermic peak top around 252°C (crystal form III); and
(iv) peaks of 6.0±0.2, 11.0±0.2, 15.4±0.2 and 16.3±0.2 and broad endotherm around 22°C to 100°C (crystal form IV).

In an embodiment, the present invention is the crystal according to the above [1] which is a crystal of the compound having a subset of the peaks at diffraction angles (2*θ* (°)) in a powder X-ray diffraction and endotherm, wherein the subset of the peaks and the endotherm are selected from the group consisting of the following (i) to (iii):
(i) peaks of 7.6±0.2, 10.1±0.2, 13.0±0.2 and 18.0±0.2 and the endothermic peak top around 266°C (crystal form I);
(ii) peaks of 11.4±0.2, 12.2±0.2, 12.5±0.2 and 17.0±0.2 and the endothermic peak top around 233°C (crystal form II); and
(iii) peaks of 10.5±0.2, 11.1±0.2, 14.1±0.2 and 24.7±0.2 and broad endotherm around 30°C to 120°C (crystal form III).

In an embodiment, the present invention relates to the crystal according to the above [1] which is a crystal of the methanesulfonic acid salt having peaks at diffraction angles (2*θ* (°)) of 7.0±0.2, 10.6±0.2, 13.7±0.2, 14.1±0.2, 16.2±0.2, 17.6±0.2, 18.6±0.2, 20.5±0.2, 21.6±0.2 and 24.5±0.2 in a powder X-ray diffraction.

In an embodiment, the present invention relates to the crystal according to the above [1] which is a crystal of the compound having peaks at diffraction angles (2*θ* (°)) of 7.6±0.2, 9.0±0.2, 10.1±0.2, 13.0±0.2, 17.1±0.2, 18.0±0.2, 19.1±0.2, 21.1±0.2, 21.7±0.2, 23.4±0.2, 26.2±0.2 and 27.5±0.2 in a powder X-ray diffraction.

In an embodiment, the present invention relates to the crystal according to the above [1] which is a crystal of the compound having peaks at diffraction angles (2*θ* (°)) of 11.4±0.2, 12.2±0.2, 12.5±0.2, 13.8±0.2, 15.0±0.2, 15.3±0.2, 17.0±0.2, 17.6±0.2, 23.1±0.2, 23.4±0.2, 24.4±0.2 and 27.1±0.2 in a powder X-ray diffraction.

### [Effect of the Invention]

The crystal of the compound or a salt thereof of the present invention has good physical properties as a drug substance.

### [Brief Description of Drawings]

Fig. 1 to Fig. 15 are powder X-ray diffraction diagrams of crystals. The vertical axis shows the diffraction intensity (Counts). The horizontal axis shows the diffraction angle (2*θ* (°)).

Fig. 16 to Fig. 30 are thermogravimetry differential thermal analysis charts (TG-DTA measurement diagrams) of crystals. The vertical axis (left) shows the mass change (%) in a thermogravimetric (TG) curve. The vertical axis (right) shows the heat flow (µV) in a differential thermal analysis (DTA) curve. The horizontal axis shows the temperature (°C).

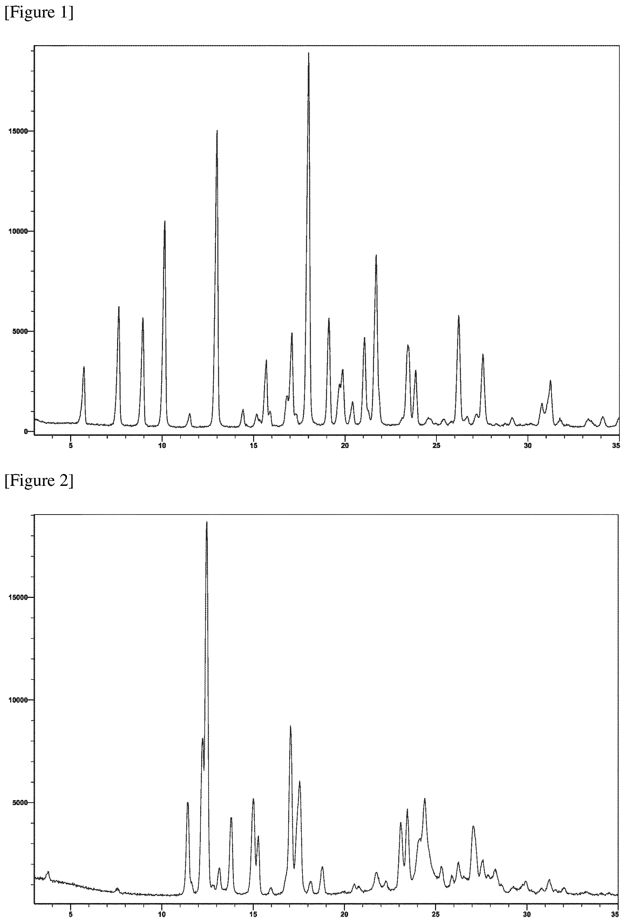

[Fig. 1] A powder X-ray diffraction diagram of crystal form I of the compound 1
[Fig. 2] A powder X-ray diffraction diagram of crystal form II of the compound 1

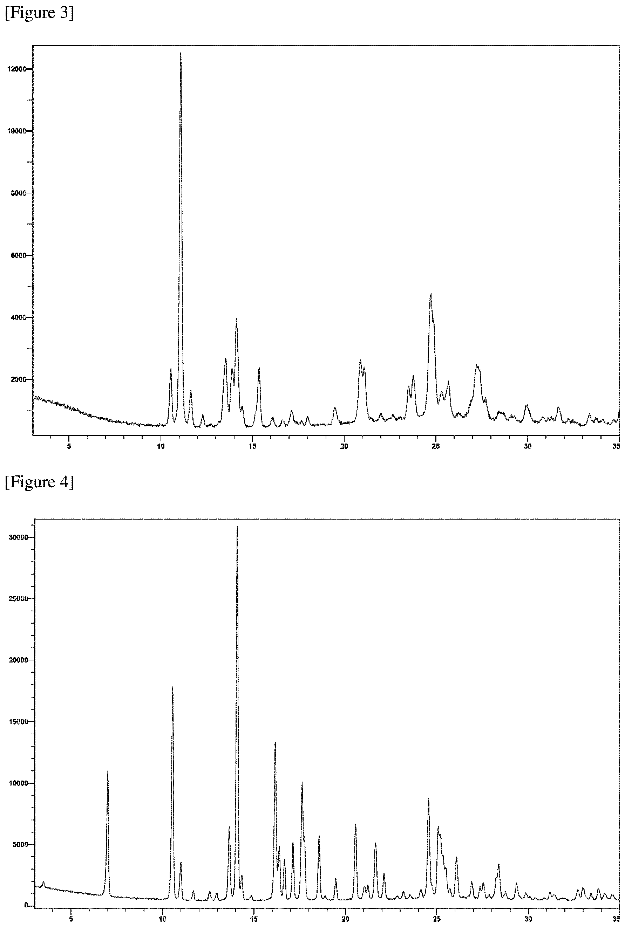

[Fig. 3] A powder X-ray diffraction diagram of crystal form III of the compound 1
[Fig. 4] A powder X-ray diffraction diagram of crystal form I of the methanesulfonic acid salt of the compound 1

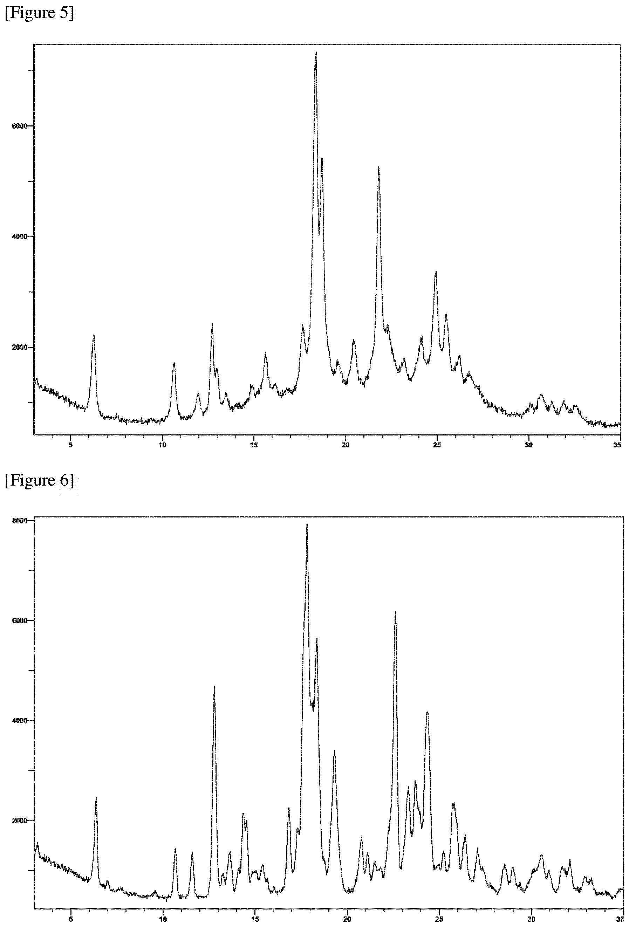

[Fig. 5] A powder X-ray diffraction diagram of crystal form II of the methanesulfonic acid salt of the compound 1
[Fig. 6] A powder X-ray diffraction diagram of crystal form III of the methanesulfonic acid salt of the compound 1

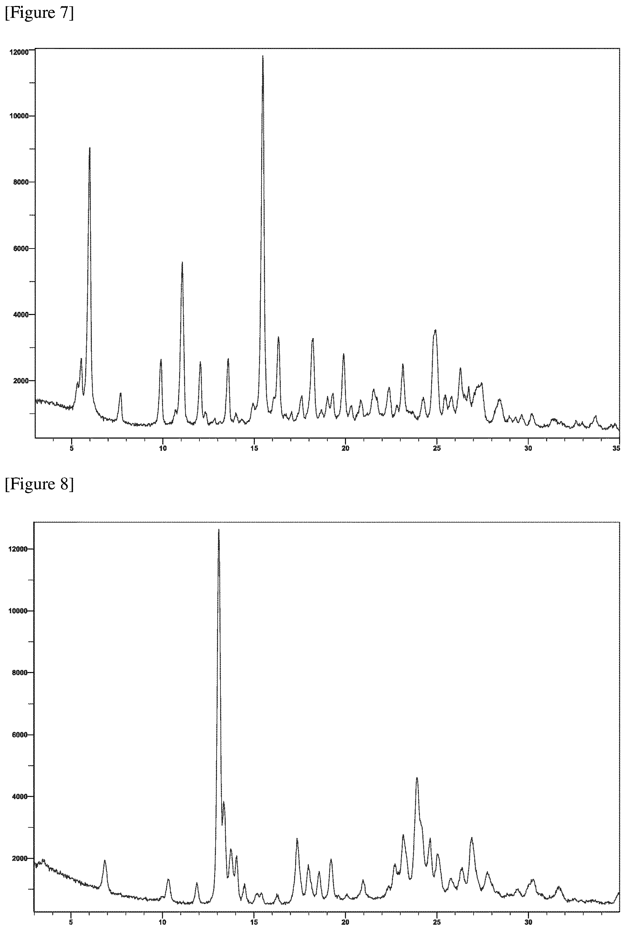

[Fig. 7] A powder X-ray diffraction diagram of crystal form IV of the methanesulfonic acid salt of the compound 1
[Fig. 8] A powder X-ray diffraction diagram of crystal form I of the hydrochloride of the compound 1

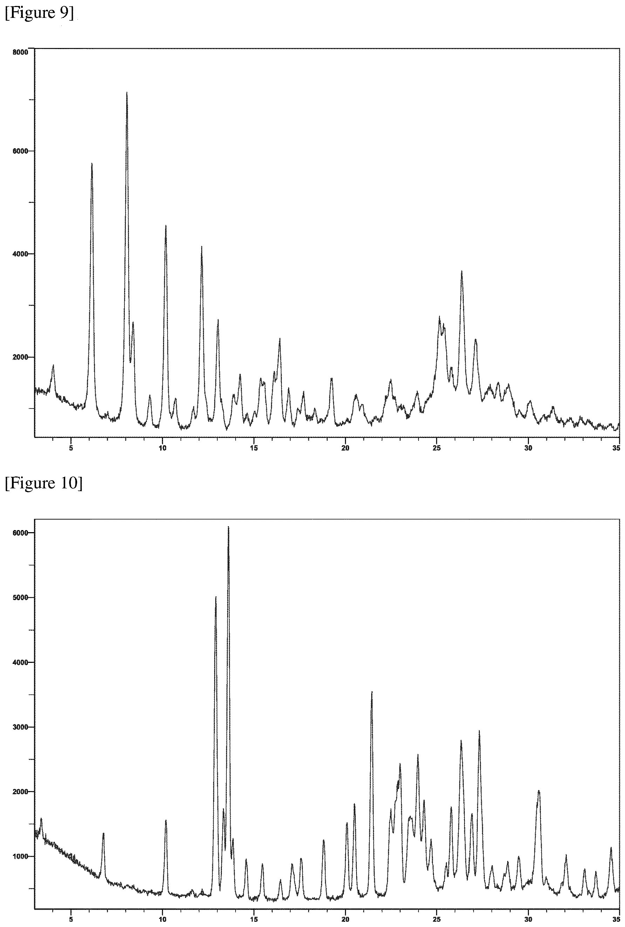

[Fig. 9] A powder X-ray diffraction diagram of crystal form II of the hydrochloride of the compound 1
[Fig. 10] A powder X-ray diffraction diagram of crystal form III of the hydrochloride of the compound 1

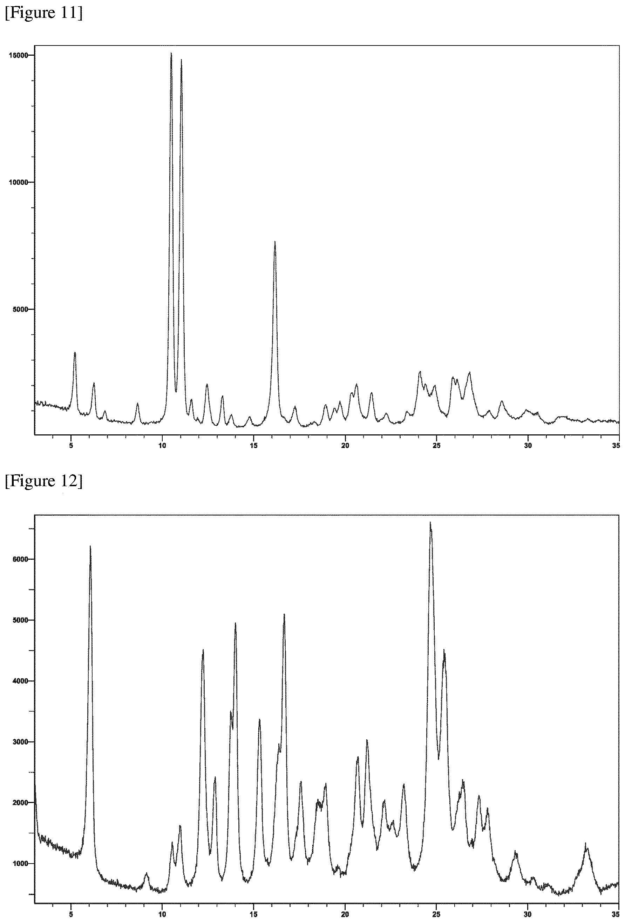

[Fig. 11] A powder X-ray diffraction diagram of crystal form IV of the hydrochloride of the compound 1
[Fig. 12] A powder X-ray diffraction diagram of crystal form I of the p-toluenesulfonic acid salt of the compound 1

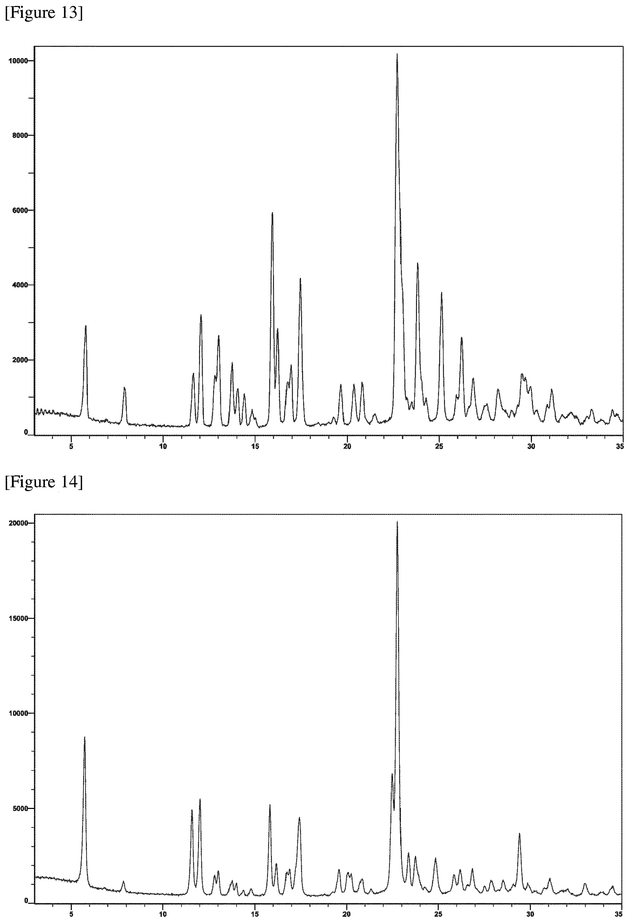

[Fig. 13] A powder X-ray diffraction diagram of crystal form I of the sodium salt of the compound 1
[Fig. 14] A powder X-ray diffraction diagram of crystal form I of the potassium salt of the compound 1

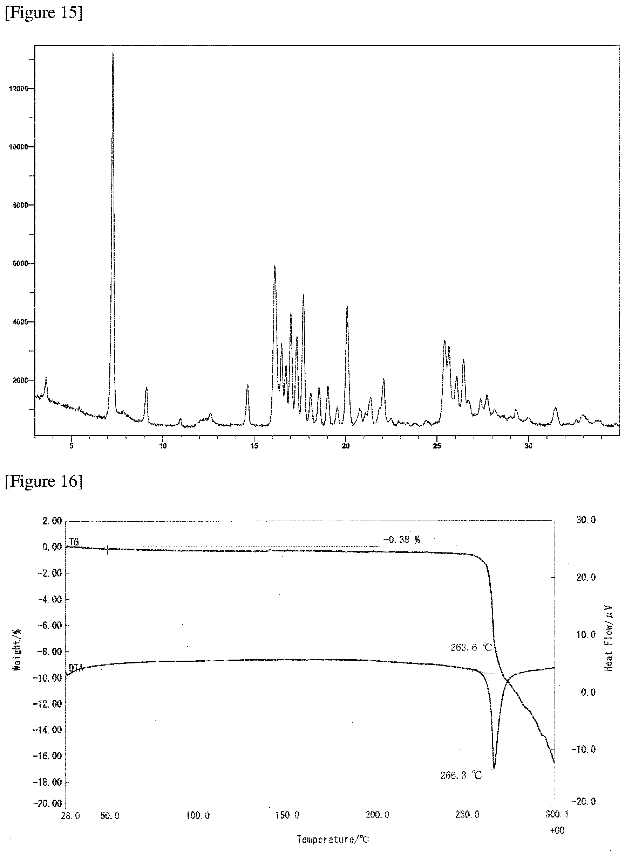

[Fig. 15] A powder X-ray diffraction diagram of crystal form I of the calcium salt of the compound 1
[Fig. 16] A TG-DTA measurement diagram of crystal form I of the compound 1

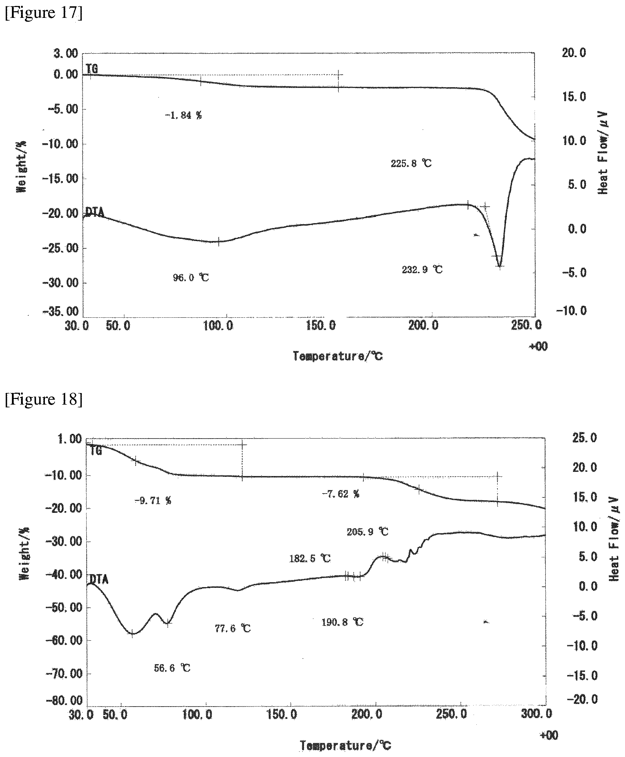

[Fig. 17] A TG-DTA measurement diagram of crystal form II of the compound 1
[Fig. 18] A TG-DTA measurement diagram of crystal form III of the compound 1

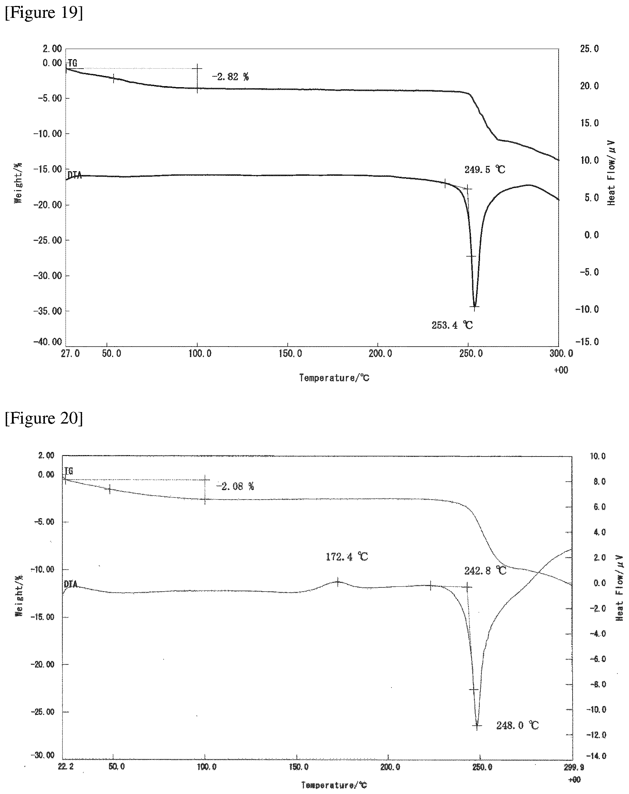

[Fig. 19] A TG-DTA measurement diagram of crystal form I of the methanesulfonic acid salt of the compound 1
[Fig. 20] A TG-DTA measurement diagram of crystal form II of the methanesulfonic acid salt of the compound 1

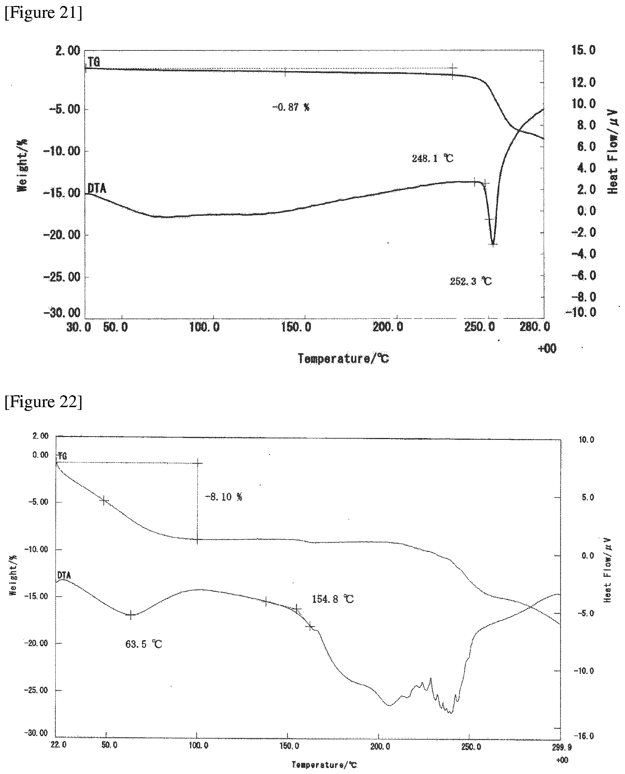

[Fig. 21] A TG-DTA measurement diagram of crystal form III of the methanesulfonic acid salt of the compound 1
[Fig. 22] A TG-DTA measurement diagram of crystal form IV of the methanesulfonic acid salt of the compound 1

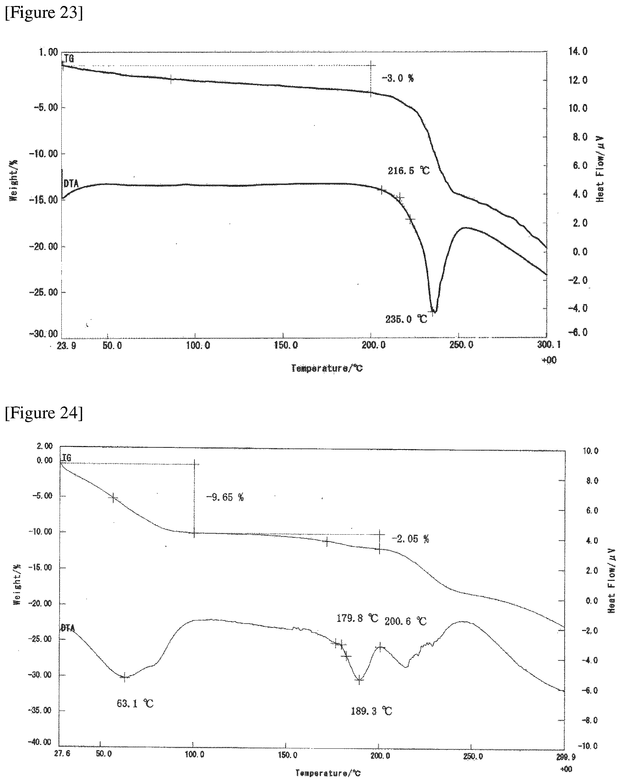

[Fig. 23] A TG-DTA measurement diagram of crystal form I of the hydrochloride of the compound 1
[Fig. 24] A TG-DTA measurement diagram of crystal form II of the hydrochloride of the compound 1

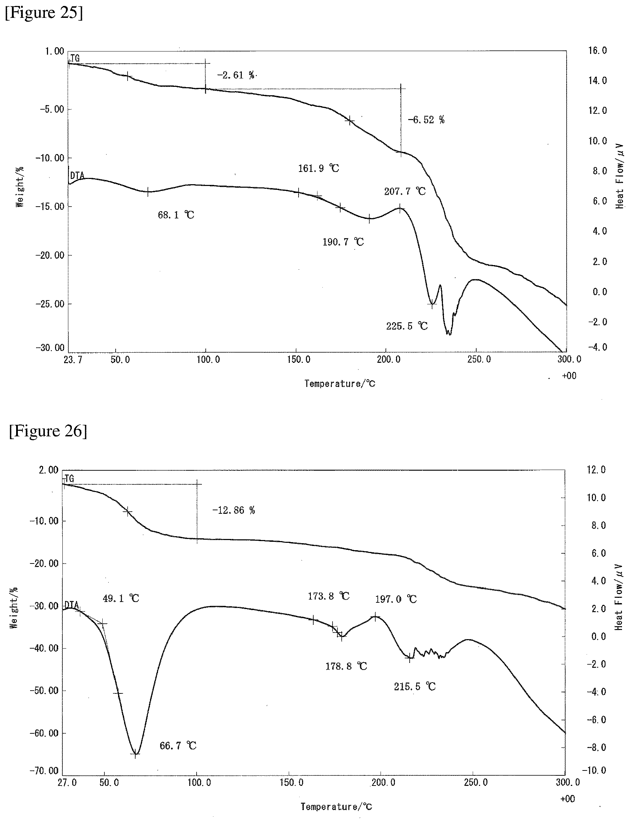

[Fig. 25] A TG-DTA measurement diagram of crystal form III of the hydrochloride of the compound 1
[Fig. 26] A TG-DTA measurement diagram of crystal form IV of the hydrochloride of the compound 1

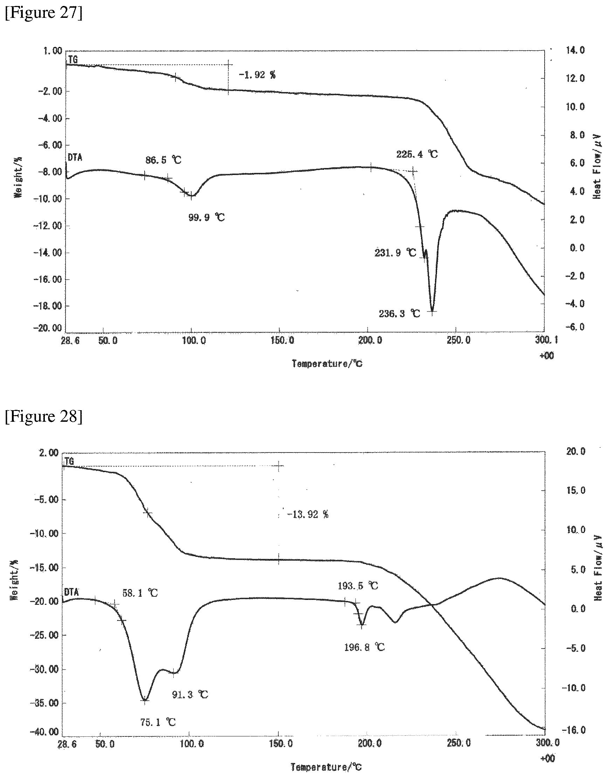

[Fig. 27] A TG-DTA measurement diagram of crystal form I of the p-toluenesulfonic acid salt of the compound 1
[Fig. 28] A TG-DTA measurement diagram of crystal form I of the sodium salt of the compound 1

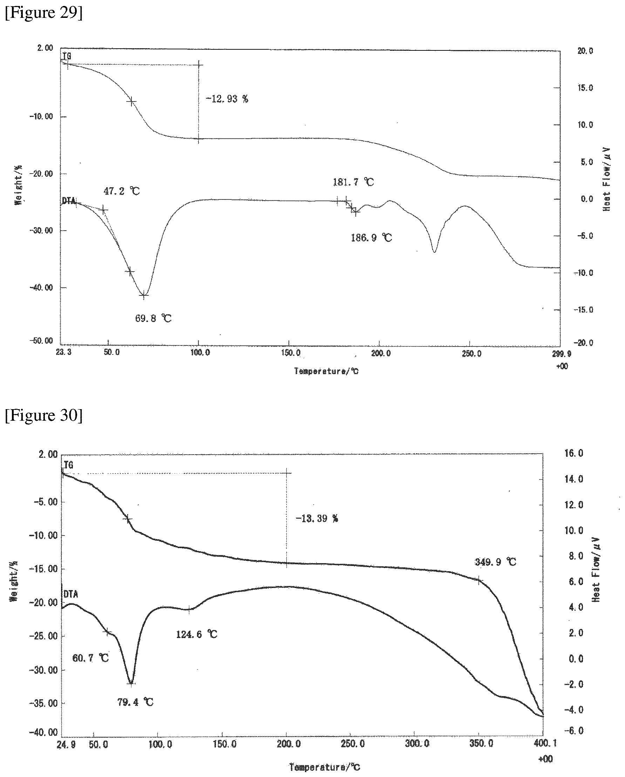

[Fig. 29] A TG-DTA measurement diagram of crystal form I of the potassium salt of the compound 1
[Fig. 30] A TG-DTA measurement diagram of crystal form I of the calcium of the compound 1

### [Mode for Carrying out the Invention]

Hereinafter, embodiments of the present invention are described in more detail.

In the present invention, each term has the following meaning unless otherwise specified.

The following abbreviations in the description, figures and tables have the following meanings, respectively.
CDI: carbonyldiimidazole
DMF: N,N-dimethylformamide
DMSO: dimethylsulfoxide
MTBE: methyl tert-butyl ether
NaBH(OAc)₃: sodium triacetoxyborohydride
NMP: 1-methyl-2-pyrrolidinone
Pd(amphos)Cl₂: bis(di-tert-butyl(4-dimethylaminophenyl)phosphine)dichloropalladium (II)
THF: tetrahydrofuran
TNBS: trinitrobenzene sulfonic acid
amino-silica gel: aminopropylated silica gel
ODS column chromatography: octadecyl-silylated silica gel column chromatography
Ex. No.: Example Number
Structure: structural formula
Physical data: physical data
IC₅₀: concentration required for 50% inhibition
FITC: Fluorescein isothiocyanate
¹H-NMR: hydrogen nuclear magnetic resonance spectrum
DMSO-d6: dimethylsulfoxide-d6
D₂O: deuterium oxide
MS: mass spectrometry
ESI_APCI: multiionization using electrospray ionization-atmospheric pressure chemical ionization

In the present invention, "as a drug substance, good physical properties" means, for example, that a crystal is physicochemically stable or chemically stable in the solid stability test shown in Test Example 4.

The compound 1 of the present invention may also be referred to as "(S)-2-((3-(1-(6-(4-carboxyphenyl)pyridin-3-yl)-2-oxo-1,2-dihydro-3H-imidazo[4,5-b]pyridin-3-yl)pyrrolidin-1-yl)methyl)-1-methyl-1H-imidazole-5-carboxylic acid".

The crystal of the compound 1 of the present invention also includes a solvate thereof with a pharmaceutically acceptable solvent such as water or ethanol. Moreover, the crystal of a salt of the compound 1 of the present invention also includes a salt, a solvate thereof with a pharmaceutically acceptable solvent such as water or ethanol, a cocrystal of the compound 1 and an appropriate conformer and a salt cocrystal (ionic cocrystal) of a salt and an appropriate coformer.

For example, the crystal form I of the methanesulfonic acid salt of the compound 1 also includes a hydrate of the methanesulfonic acid salt. The number of water molecules incorporated into the crystal lattice may vary from 0 to 1 depending on the humidity, and the crystal form I of the methanesulfonic acid salt of the compound 1 also includes any hydrate.

In the compound 1 of the present invention, part of the atoms may be replaced with corresponding isotopes. The present invention includes compounds in which atoms are replaced with these isotopes. Examples of the isotopes include isotopes of a hydrogen atom, a carbon atom, a chlorine atom, a fluorine atom, an iodine atom, a nitrogen atom, an oxygen atom, a phosphorus atom and a sulfur atom represented by ²H, ³H, ¹¹C, ¹³C, ¹⁴C, ³⁶Cl , ¹⁸F, ¹²³I, ¹²⁵I, ¹³N, ¹⁵N, ¹⁵O,¹⁷O, ¹⁸O, ³²P and ³⁵S. In an embodiment, a compound in which part of the hydrogen atoms of the compound 1 are replaced with ²H (D: deuterium atoms) can be illustrated.

The compound 1 of the present invention in which part of the atoms are replaced with isotopes can be prepared by a similar method to the method for manufacturing described below using a commercial isotope-introduced building block.

The compound 1 of the present invention has an excellent PHD2 inhibitory effect and thus can be used as a therapeutic agent for IBD (see, Nature Reviews Drug Discovery, 2014, 13, pp. 852-869). In the present invention, the phrase "IBD" includes, for example, ulcerative colitis, Crohn's disease, intestinal Behcet disease, infectious enteritis, radiation enteritis, drug-induced enteritis, ischemic enteritis, mesenteric phlebosclerosis (phlebosclerotic colitis), obstructive colitis and enteritis due to collagen disease. Preferably, the compound 1 of the present invention can be used as a therapeutic agent for ulcerative colitis or Crohn's disease (see, Inflamm. Bowel. Dis., 2015, 21 (2), pp. 267-275).

In the present invention, the phrase "treatment" includes the meanings of "prevention". The treatment of ulcerative colitis includes, for example, the meanings of "prevention of relapse" and "maintenance of remission".

The therapeutic effects on colitis of the compound 1 of the present invention can be determined according to the method described in Test Example 2 or well-known methods in the technical field. For example, the effects can also be determined according to the method described in Biol. Pharm. Bull., 2004, 27 (10), pp.1599-1603 and the like or similar methods thereto.

In an embodiment, the compound 1 of the present invention is a PHD2 inhibitor that acts specifically on the large intestine tissue to limit the off-target effects of stabilization of HIF-α. The term "acts specifically on the large intestine tissue" means, for example, that the concentration of the compound is high in the large intestine tissue compared to that in the blood and that the compound exerts a therapeutic effect on large intestine without systemic effects (for example, hematopoietic effect) (see, Test Examples 2 and 3).

The pharmaceutical composition of the present invention is used in various dosage forms depending on the usage. As such dosage forms, for example, powders, granules, fine granules, dry syrups, tablets, capsules, injections, liquids, ointments, suppositories, poultices and enema agents can be illustrated. Preferably, the pharmaceutical composition of the present invention is orally administered.

The pharmaceutical composition of the present invention comprises a crystal of the compound 1 or a salt thereof as an active ingredient.

The pharmaceutical composition of the present invention is prepared using a crystal of the compound 1 or a salt thereof and at least one pharmaceutical additive. These pharmaceutical compositions can be formulated by appropriately admixing, diluting or dissolving with appropriate pharmaceutical additives such as excipients, disintegrants, binders, lubricants, diluents, buffers, tonicity agents, preservatives, wetting agents, emulsifying agents, dispersing agents, stabilizing agents, solubilizing agents and the like, according to a known formulation procedure depending upon their dosage forms.

When the pharmaceutical composition of the present invention is used in the treatment, the dosage of the compound 1 or the salt thereof is appropriately decided depending on the age, sex, body weight and degree of disorders and treatment of each patient and the like. The daily dose can be divided into one, two, three or four times per day and administered.

The dosage for an adult can be decided within the range of, for example, 0.1 to 1000 mg per day in the case of oral administration. In an embodiment, the oral administration dosage can be decided within the range of 1 to 500 mg per day and is preferably within the range of 10 to 200 mg per day.

The dosage for an adult can be decided at, for example, 0.1 to 1000 mg per day in the case of parenteral administration. In an embodiment, the parenteral administration dosage can be decided within the range of 0.5 to 200 mg per day and is preferably within the range of 1 to 20 mg per day.

In an embodiment, the pharmaceutical composition of the present invention can also be used in combination with any other medicament other than PHD inhibitors. As such other medicaments which can be used in combination for the treatment of inflammatory bowel diseases, for example, 5-ASA, steroids, immunosuppressive agents, TNF-α antibodies, Janus kinase inhibitors and α4β7 integrin antibodies can be illustrated.

When the crystal of the compound 1 or a salt thereof of the present invention is used in combination with the other medicament, they can be administered as a formulation comprising these active ingredients or as formulations which are each separately formulated from each active ingredient. When separately formulated, these formulations can be administered separately or concurrently. Furthermore, the dosage of the compound 1 of the present invention or a salt thereof can be appropriately reduced depending on the dosage of the other medicament used in combination.

### Powder X-ray Diffraction Measurement

For the powder X-ray diffraction, the crystals were ground with a mortar and then measured with a powder X-ray diffraction apparatus SmartLab (Rigaku) by reflection method according to the following conditions.
X-ray source, Wavelength: CuKα rays (CuKα1 and CuKα2), 1.5418Å
Tube voltage, Tube current, Scanning speed: 40 kV, 50 mA, 13° 2(*θ*)/min
Data analysis software: SmartLab Studio II (Rigaku)
Data analysis method (peak definition): Peak position (peak top position, diffraction angles upon irradiation with CuKα1 and CuKα2), peak height (exclude background)

It is common knowledge that the relative intensity of a peak (relative peak height) in powder X-ray diffraction patterns may fluctuate depending on the sample conditions and the measurement conditions. The relative intensity can slightly vary depending on the direction of crystal growth, the size of particles, the measurement conditions or the like and therefore should not be strictly interpreted.

It is also common knowledge that the 2*θ* value of each peak in powder X-ray diffraction may slightly fluctuate depending on the sample conditions and the measurement conditions. In general, the 2*θ* values may fluctuate within a range of about ±0.2 (°). Therefore, the present invention encompasses not only crystals in which the diffraction angles (2*θ* (°)) of peaks in powder X-ray diffraction completely coincide but also crystals in which the diffraction angles (2*θ* (°)) of all or a part of the peaks coincide within a range of ±0.2 (°).

### Thermal Analysis Measurement (Thermogravimetric differential thermal analysis (TG-DTA))

Thermal analysis was performed using a differential thermobalance (type TG8120 or type Thermo plus EVO2, Rigaku) under nitrogen atmosphere according to the following measurement conditions.
Heating rate: 10°C/min
Reference material: Aluminium oxide

The temperature at which the mass decrease started and the temperature at which it converged were measured, and the mass change (%) was calculated from the mass difference at the temperatures.

In a TG-DTA measurement diagram, "Endotherm" in a DTA curve is represented by the temperature at peak top (peak top) or "extrapolation initiation temperature". "Extrapolated start temperature" means the intersection between the onset point or the offset point in the DTA curve and extrapolation of the baseline and is also referred to as "extrapolated onset temperature". "Extrapolated start temperature" is a temperature at the starting point of the peak, and it means exothermic or endothermic starting temperature calculated by the extrapolation. The peak top and the extrapolated start temperature in a TG-DTA measurement diagram may slightly fluctuate depending on the measurement conditions. For example, in general, the temperature may fluctuate within a range of ±5°C. Thus, the crystal specified by the above peaks encompasses crystals which coincide within a range of ±5°C.

In the present invention, "around" used in the thermal analysis means a range of ±5°C.

The crystal of the compound 1 or a salt thereof of the present invention can be specified for its crystal form by a subset of the diffraction peaks in the powder X-ray diffraction described in each example. In the present invention, "having a subset of the peaks" means that a crystal includes at least the peaks of the subset as the characteristic peaks.

Further, crystal forms can also be specified by a combination of a subset of the diffraction peaks in the powder X-ray diffraction and physical properties such as endotherm in TG-DTA measurement of each crystal.

### [Examples]

The present invention is further illustrated in more detail by way of the following Examples. However, the present invention is not limited thereto.

### Reference Example 1

### tert-Butyl (S)-3-((3-nitropyridin-2-yl)amino)pyrrolidine-1-carboxylate

To NMP (100 mL) were added 2-fluoro-3-nitropyridine (10.00 g), tert-butyl (S)-3-aminopyrrolidine-1-carboxylate (13.10 g) and potassium carbonate (11.67 g) under ice-cooling. The reaction mixture was stirred at 150°C for 1 hour. The reaction mixture was allowed to cool to room temperature. To the reaction mixture were added ethyl acetate and water, and the resultant was mixed. The mixture was extracted with ethyl acetate. The organic layer was washed with water and brine, then dried over anhydrous magnesium sulfate added thereto and concentrated under reduced pressure to give the title compound (21.70 g).

### Reference Example 2

### tert-Butyl (S)-3-((3-aminopyridin-2-yl)amino)pyrrolidine-1-carboxylate

To a mixture of Reference Example 1 (21.70 g) and ethanol (300 mL) was added 10% palladium on carbon (2.17 g, wet) under an argon atmosphere. The mixture was stirred under a hydrogen atmosphere at room temperature for 6 hours. The reaction mixture was filtered through Celite, and the filtrate was concentrated under reduced pressure to give the title compound (19.74 g).

### Reference Example 3

### tert-Butyl (S)-3-(2-oxo-1,2-dihydro-3H-imidazo[4,5-b]pyridin-3-yl)pyrrolidine-1-carboxylate

To a mixture of Reference Example 2 (19.59 g) and THF (200 mL) was added CDI (22.82 g) under ice-cooling with stirring. The reaction mixture was stirred at room temperature for 30 minutes. To the reaction mixture was added an aqueous solution of sodium hydroxide (5 mol/L, 30 mL), and the resulting mixture was stirred for 10 minutes. To the reaction mixture was added hydrochloric acid (2 mol/L, 75 mL). After stirring, the mixture was concentrated under reduced pressure. To the obtained mixture was added water, and the mixture was extracted with ethyl acetate. The organic layer was washed with brine, then dried over anhydrous magnesium sulfate and concentrated under reduced pressure. The obtained residue was purified by column chromatography on silica gel (eluent: n-hexane/ethyl acetate=50/50-0/100) to give the title compound (17.61 g). MS (ESI_APCI, m/z): 303 (M-H)⁻

### Reference Example 4

### tert-Butyl (S)-3-(1-(6-chloropyridin-3-yl)-2-oxo-1,2-dihydro-3H-imidazo[4,5-b]pyridin-3-yl)pyrrolidine-1-carboxylate

A mixture of Reference Example 3 (100 mg), 2-chloro-5-iodopyridine (94 mg), N,N' -dimethylethylenediamine (0.042 mL), copper (I) iodide (75 mg), potassium carbonate (136 mg) and acetonitrile (3 mL) was stirred at 100°C under microwave irradiation for 1 hour. The reaction mixture was poured into a mixture of water and hydrochloric acid (1 mol/L). After stirring, the mixture was extracted with ethyl acetate. The organic layer was washed with brine, then dried over anhydrous magnesium sulfate and concentrated under reduced pressure. The obtained residue was purified by column chromatography on silica gel (eluent: n-hexane/ethyl acetate=35/65) to give the title compound (117 mg).

### Reference Example 5

### tert-Butyl (S)-3-(1-(6-(4-(methoxycarbonyl)phenyl)pyridin-3-yl)-2-oxo-1,2-dihydro-3H-imidazo[4,5-b]pyridin-3-yl)pyrrolidine-1-carboxylate

A mixture of Reference Example 4 (117 mg), (4-(methoxycarbonyl)phenyl)boronic acid (61 mg), Pd(amphos)Cl₂ (20 mg), sodium carbonate (72 mg), DMF (3 mL) and water (0.3 mL) was stirred at 150°C under microwave irradiation for 1 hour. The reaction mixture was poured into a mixture of water and ethyl acetate. After stirring, the mixture was extracted with ethyl acetate. The organic layer was washed with water and brine, then dried over anhydrous magnesium sulfate and concentrated under reduced pressure. The obtained residue was purified by column chromatography on silica gel (eluent: n-hexane/ethyl acetate=80/20-30/70) to give the title compound (95 mg).

### Reference Example 6

### Methyl (S)-4-(5-(2-oxo-3-(pyrrolidin-3-yl)-2,3-dihydro-1H-imidazo[4,5-b]pyridin-1-yl)pyridin-2-yl)benzoate hydrochloride

A mixture of Reference Example 5 (95 mg), hydrogen chloride-1,4-dioxane solution (4 mol/L, 1 mL) and methanol (1 mL) was stirred at room temperature for 30 minutes. The reaction mixture was concentrated under reduced pressure to give the title compound (126 mg).

### Reference Example 7

### Methyl (S)-4-(5-(3-(1-((5-bromo-1-methyl-1H-imidazol-2-yl)methyl)pyrrolidin-3-yl)-2-oxo-2,3-dihydro-1H-imidazo[4,5-b]pyridin-1-yl)pyridin-2-yl)benzoate

To a mixture of Reference Example 6 (200 mg), 5-bromo-1-methyl-1H-imidazole-2-carbaldehyde (167 mg) and dichloromethane (2 mL) was added NaBH(OAc)₃ (375 mg). The reaction mixture was stirred at room temperature for 30 minutes. To the reaction mixture was added methanol (2 mL), and the mixture was stirred for 30 minutes. The reaction mixture was concentrated under reduced pressure. The obtained residue was purified by column chromatography on amino-silica gel (eluent: n-hexane/ethyl acetate=80/20-0/100) to give the title compound (214 mg). MS (ESI_APCI, m/z): 588 (M+H)⁺

### Reference Example 8

### Methyl (S)-4-(5-(3-(1-((5-cyano-1-methyl-1H-imidazol-2-yl)methyl)pyrrolidin-3-yl)-2-oxo-2,3-dihydro-1H-imidazo[4,5-b]pyridin-1-yl)pyridin-2-yl)benzoate

A mixture of Reference Example 7 (214 mg), tetrakis(triphenylphosphine)palladium (0) (42 mg), zinc cyanide (86 mg) and NMP (3 mL) was stirred at 150°C under microwave irradiation for 1 hour. The reaction mixture was poured into a mixture of ethyl acetate and water. After stirring, the mixture was extracted with ethyl acetate. The organic layer was washed with brine, then dried over anhydrous magnesium sulfate and concentrated under reduced pressure. The obtained residue was purified by column chromatography on amino-silica gel (eluent: n-hexane/ethyl acetate=80/20-0/100) to give the title compound (244 mg). MS (ESI_APCI, m/z): 535 (M+H)⁺

### Compound 1

### 2-{[(3S)-3-{1-[6-(4-Carboxyphenyl)pyridin-3-yl]-2-oxo-2,3-dihydro-1H-imidazo[4,5-b]pyridin-3-yl}pyrrolidin-1-yl]methyl}-1-methyl-1H-imidazole-5-carboxylic acid

A mixture of Reference Example 8 (195 mg) and concentrated hydrochloric acid (1 mL) was stirred at 110°C under microwave irradiation for 1 hour. The reaction mixture was neutralized with the addition of an aqueous solution of sodium hydroxide (5 mol/L). The mixture was diluted with DMSO added thereto. Then, the insoluble material was removed through Celite. The filtrate was purified by ODS column chromatography (eluent: water/acetonitrile=98/2-30/70) to give the title compound (60 mg).

**[Table 1]**

| | Structure | Physical data | IC₅₀ (*µ*M) |
|---|---|---|---|
| Compound 1 | 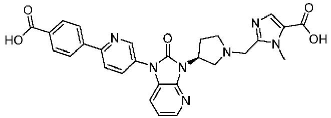 | ¹H-NMR(DMSO-d6) δ ppm: 2.15-2.30 (1H, m), 2.40-2.56 (1H, m), 2.82-3.04 (4H, m), 3.82 (2H, s), 3.91 (3H, s), 5.05-5.17 (1H, m), 7.13 (1H, dd, J=5.2, 7.8Hz), 7.49 (1H, s), 7.56 (1H, dd, J=1.3. 7.8Hz), 8.06-8.11 (2H, m), 8.12 (1H, dd, J=1.3, 5.2Hz), 8.18 (1H, dd, J=2.6, 8.6Hz), 8.25-8.32 (3H, m), 8.95 (1H, dd, J=0.5, 2.6Hz), 12.95 (2H, brs) | 0.18 |

### Test Example 1 PHD2 inhibitory test

### (1) Expression and preparation of human PHD2₁₈₄₋₄₁₈

Human PHD2₁₈₄₋₄₁₈ containing amino acid residues 184 to 418 of the protein represented by CAC42509 (GenBank accession ID) was expressed and prepared by the following method.

An expression construct of human PHD2₁₈₄₋₄₁₈ containing an N-terminal histidine tag was introduced into pET-30a (+) vector, and the sequence was confirmed. This vector was introduced into BL21 (DE3) strain and cultured at 37°C in LB medium containing antibiotics. After culturing, a cell lysis solution was added to the cells, and then the cells were disrupted and suspended by sonication. The disrupted suspension was centrifuged, and the supernatant was purified by Ni column to give human PHD2₁₈₄₋₄₁₈.

### (2) Test methods

Human HIF-1α₅₅₆₋₅₇₄ (FITC-labeled HIP-1α₅₅₆₋₅₇₄), containing FITC-Ahx at the N-terminal of HIP-1α₅₅₆₋₅₇₄ containing amino acid residues 556 to 574 (partial peptide) of human HIF-1α, was used as a substrate. Using FITC-labeled HIF-1α₅₅₆₋₅₇₄, the competitive inhibition between 2-oxoglutarate and the test compound (PHD inhibitor) was evaluated based on the change in fluorescence polarization of FITC-labeled HIF-1α₅₅₆₋₅₇₄ by the following method.

An enzyme (human PHD2₁₈₄₋₄₁₈) and the substrate were diluted with an assay buffer (pH 7.4) containing 10 mM HEPES, 150 mM NaCl, 10 µM MnCl₂-4H₂O, 2 µM 2-oxoglutarate and 0.05% Tween-20. The test compound was diluted with DMSO. The test compound and human PHD2₁₈₄₋₄₁₈ were added to the 384-well plate (Corning, black, opaque bottom) in advance. The reaction was started by the addition of FITC-labeled HIF-1α₅₅₆₋₅₇₄. After incubating at 37°C for 60 minutes, fluorescence polarization (excitation wavelength: 470 nm, fluorescence wavelength: 530 nm) was measured by PHERAstar FSX (BMG Labtech). The fluorescence polarization of each well was measured, and the human PHD2 binding inhibitory activity of the test compound was calculated based on the value of the test substance-free group.

### (3) Results

As shown in table 1, the compound 1 of the present invention inhibited binding between PHD2 and HIF-1α. Thus, it was demonstrated that the compound 1 of the present invention is useful as a PHD2 inhibitor.

### Test Example 2 Therapeutic effect in colitis model

### (1) TNBS induced colitis model rat

It is known that inflammation is locally caused in the large intestine when TNBS is administered into the large intestine of a rat and that the intestinal permeability is then increased due to breakdown of barrier function in the intestines. Thus, the suppressive effect on the intestinal permeability based on oral administration of the test compound was evaluated as an indicator of medicinal efficacy.

### (2) Test methods

SD male rats: 8-week-old SLC (Japan SLC) were used. Under pentobarbital anesthesia, 300 µL of 28 mg/mL TNBS which was adjusted with 50% ethanol was administered at a point 8 cm from the anus in the large intestine to cause inflammation. To the solvent-treated group was administrated 300 µL of 50% ethanol. Animals were fasted for 48 hours prior to administration of TNBS. The test compound (3 mg/kg) prepared with 0.05% methylcellulose solution was orally administered once a day from the next day, and it was administered for a total of 3 days. After administering for 3 days, 50 mg/kg FITC was orally administered 4 hours after the administration of the test compound. Blood samples were collected from jugular vein under isoflurane anesthesia after 4 hours. The serum was centrifuged, and fluorescence intensity was detected by PHERAstar FSX( BMG Labtech) to measure the concentration of FITC permeating into circulating blood through the mesentery. The suppressive rate on the intestinal permeability of the test compound was calculated based on the value of the test substance-free group as 0 and the value of the TNBS-untreated group as 100.

### (3) Results

The suppressive rate (%, mean) on the intestinal permeability of the test compound (Inhibition) is shown below.

**[Table 2]**

| | Inhibition (%) |
|---|---|
| Compound 1 | 101 |

The intestinal permeability of FITC, which was increased due to the administration of TNBS, was suppressed by the administration of the compound 1 of the present invention. Thus, it was demonstrated that the compound 1 of the present invention is useful as an agent for the treatment of inflammatory bowel diseases.

### Test Example 3 Concentration of compound in large intestine tissue

### (1) Rat PK study

The test compound (3 mg/kg/5 mL) prepared with 0.05% methylcellulose was orally administered to non-fasted rats (SD, 8-week-old, male, Japan SLC). Blood samples were collected from jugular vein 0.25, 0.5, 1, 2, 4, 6 and 8 hours after the administration. Laparotomy was performed under isoflurane anesthesia, and the large intestine was isolated. The collected distal large intestine (about 5 cm) was cut open, and then the large intestine extracted was washed with saline on a dish. After washing, the large intestine was minced with small scissors. About 150 mg thereof was moved to a tube. To the tube was added 100 µL of saline, and the mixture was homogenized using shake master (1000 rpm × 30 minutes). Samples were prepared by the addition with quadruple volume of saline as the final volume. The concentrations of the test compound in the large intestine tissue and the plasma were measured through a quantitative analysis using liquid chromatography-mass spectrometry (LC/MS).

### (2) Concentrations of compound in large intestine tissue and plasma

As shown in the following table, it was demonstrated that the compound 1 of the present invention has a higher concentration in the large intestine tissue than the concentration in the plasma. Accordingly, the compound 1 of the present invention is a PHD2 inhibitor that acts specifically on the large intestine tissue.

**[Table 3]**

| | Cmax | AUC | Plasma | Colon | C/P |
|---|---|---|---|---|---|
| Compound 1 | 1 | 117 | <1 | 164 | >164 |

Symbols in the table have the following meanings.
Cmax: maximum plasma concentration of the test compound in the case of oral administration (ng/mL)
AUC: area under the plasma test compound concentration-time curve (ng^{∗}min/mL) Plasma: plasma test compound concentration after 8 hours (ng/mL)
Colon: concentration of the test compound in the large intestine tissue after 8 hours (ng/g)
C/P: ratio of the above Colon and Plasma

### Example 1-1: crystal form I of the compound 1

The compound 1 (100 mg) was added to DMSO (1 mL) and dissolved at 60°C, and the mixture was passed through a glass filter. DMSO was removed from the filtrate with a centrifugal evaporator. To the residue in the vessel was added ethanol (2 mL). The mixture was stirred for 1 hour at room temperature, and the slurry was filtered under suction. The obtained solid was washed with water (1 mL) one time and ethanol (2 mL) twice in this order. The obtained solid was dried under reduced pressure for 1 day at 50°C to give the crystal form I of the compound 1 (66 mg).

The powder X-ray diffraction for the crystal form I of the compound 1 was measured. The diffraction angles (2*θ* (°)) of major diffraction peaks and the relative intensities (Rel. Den. (%)) of the diffraction peaks are shown in table 4.

**[Table 4]**

| *2 θ* (° ) | Rel. Den.(%) | *2 θ* (° ) | Rel. Den.(%) |
|---|---|---|---|
| 7.6 | 32 | 19.1 | 29 |
| 9.0 | 29 | 21.1 | 24 |
| 10.1 | 56 | 21.7 | 42 |
| 13.0 | 81 | 23.4 | 23 |
| 17.1 | 24 | 26.2 | 30 |
| 18.0 | 100 | 27.5 | 19 |

For the identification of the crystal form I of the compound 1, for example, a subset of the diffraction peaks selected from the group consisting of the following [1-1-1] to [1-1-7] can be used:
[1-1-1] peaks of 10.1±0.2 and 18.0±0.2;
[1-1-2] peaks of 7.6±0.2 and 18.0±0.2;
[1-1-3] peaks of 10.1±0.2, 13.0±0.2 and 18.0±0.2;
[1-1-4] peaks of 7.6±0.2, 13.0±0.2 and 18.0±0.2;
[1-1-5] peaks of 7.6±0.2, 10.1±0.2, 13.0±0.2 and 18.0±0.2;
[1-1-6] peaks of 7.6±0.2, 9.0±0.2, 10.1±0.2, 13.0±0.2 and 18.0±0.2; and
[1-1-7] peaks of 7.6±0.2, 9.0±0.2, 10.1±0.2, 13.0±0.2, 17.1±0.2, 18.0±0.2, 19.1±0.2, 21.1±0.2, 21.7±0.2, 23.4±0.2, 26.2±0.2 and 27.5±0.2.

Thermal analysis for the crystal form I of the compound 1 was performed. Endotherm: around 266°C (peak top, extrapolated start temperature around 264°C) Mass decrease: around 28°C to 200°C (0.4%)

### Example 1-2: crystal form II of the compound 1

To a mixture of the compound 1 (1.00 g), ethanol (3.8 mL) and water (12 mL) was added an aqueous solution of 2 mol/L sodium hydroxide (1.85 mL) at room temperature. After dissolution, the solution was passed through a glass filter. The filtrate was stirred for 40 minutes at 64°C. To the solution was added 2 mol/L hydrochloric acid (0.925 mL) at the same temperature. After stirring for 20 minutes at the same temperature, to the mixture was added 2 mol/L hydrochloric acid (0.463 mL). After further stirring for 1 hour, to the mixture was added 2 mol/L hydrochloric acid (0.463 mL). After stirring the mixture for 1 day at room temperature, the slurry was filtered under suction. The obtained solid was washed with water (10 mL) twice and ethanol (10 mL) in this order. The obtained solid was dried under reduced pressure for 4 days at 50°C to give the crystal form II of the compound 1 (940 mg).

The powder X-ray diffraction for the crystal form II of the compound 1 was measured. The diffraction angles (2*θ* (°)) of major diffraction peaks and the relative intensities (Rel. Den. (%)) of the diffraction peaks are shown in table 5.

**[Table 5]**

| 2 *θ* (° ) | Rel. Den.(%) | 2 *θ* (° ) | Rel. Den.(%) |
|---|---|---|---|
| 11.4 | 25 | 17.0 | 43 |
| 12.2 | 38 | 17.6 | 28 |
| 12.5 | 100 | 23.1 | 17 |
| 13.8 | 22 | 23.4 | 19 |
| 15.0 | 25 | 24.4 | 19 |
| 15.3 | 15 | 27.1 | 15 |

For the identification of the crystal form II of the compound 1, for example, a subset of the diffraction peaks selected from the group consisting of the following [1-2-1] to [1-2-7] can be used:
[1-2-1] peaks of 12.5±0.2 and 17.0±0.2;
[1-2-2] peaks of 11.4±0.2 and 17.0±0.2;
[1-2-3] peaks of 11.4±0.2, 12.5±0.2 and 17.0±0.2;
[1-2-4] peaks of 12.2±0.2, 12.5±0.2 and 17.0±0.2;
[1-2-5] peaks of 11.4±0.2, 12.2±0.2, 12.5±0.2 and 17.0±0.2;
[1-2-6] peaks of 11.4±0.2, 12.2±0.2, 12.5±0.2, 17.0±0.2 and 17.6±0.2; and
[1-2-7] peaks of 11.4±0.2, 12.2±0.2, 12.5±0.2, 13.8±0.2, 15.0±0.2, 15.3±0.2, 17.0±0.2, 17.6±0.2, 23.1±0.2, 23.4±0.2, 24.4±0.2 and 27.1±0.2.

Thermal analysis for the crystal form II of the compound 1 was performed.
Endotherm: around 96°C (broad endotherm at 50 to 120°C), around 233°C (peak top, extrapolated start temperature around 226°C)
Mass decrease: around 30°C to 150°C (1.8%)

### Example 1-3: crystal form III of the compound 1

To a mixture of the compound 1 (150 mg), 2-propanol (3 mL) and water (1.5 mL) was added an aqueous solution of 2 mol/L sodium hydroxide (0.280 mL) at room temperature. After dissolution, to the solution was slowly added dropwise a mixture solution of 2 mol/L hydrochloric acid (0.280 mL), 2-propanol (0.200 mL) and water (0.200 mL) at 50°C. The mixture was stirred for 2 hours at the same temperature and stirred for 2 hours at room temperature. The slurry was filtered under suction. The obtained solid was dried under reduced pressure for 15 hours at room temperature to give the crystal form III of the compound 1 (135 mg).

The powder X-ray diffraction for the crystal form III of the compound 1 was measured. The diffraction angles (2*θ* (°)) of major diffraction peaks and the relative intensities (Rel. Den. (%)) of the diffraction peaks are shown in table 6.

**[Table 6]**

| *2 θ* (° ) | Rel. Den.(%) | 2 *θ* (° ) | Rel. Den.(%) |
|---|---|---|---|
| 10.5 | 15 | 15.4 | 16 |
| 11.1 | 100 | 20.9 | 16 |
| 11.6 | 9 | 21.1 | 12 |
| 13.5 | 17 | 23.8 | 10 |
| 13.9 | 14 | 24.7 | 17 |
| 14.1 | 28 | | |

For the identification of the crystal form III of the compound 1, for example, a subset of the diffraction peaks selected from the group consisting of the following [1-3-1] to [1-3-7] can be used:
[1-3-1] peaks of 11.1±0.2 and 14.1±0.2;
[1-3-2] peaks of 10.5±0.2 and 11.1±0.2;
[1-3-3] peaks of 10.5±0.2, 11.1±0.2 and 14.1±0.2;
[1-3-4] peaks of 11.1±0.2, 14.1±0.2 and 24.7±0.2;
[1-3-5] peaks of 10.5±0.2, 11.1±0.2, 14.1±0.2 and 24.7±0.2;
[1-3-6] peaks of 10.5±0.2, 11.1±0.2, 13.5±0.2, 14.1±0.2 and 24.7±0.2; and
[1-3-7] peaks of 10.5±0.2, 11.1±0.2, 11.6±0.2, 13.5±0.2, 13.9±0.2, 14.1±0.2, 15.4±0.2, 20.9±0.2, 21.1±0.2, 23.8±0.2 and 24.7±0.2.

Thermal analysis for the crystal form III of the compound 1 was performed.
Endotherm: around 57°C and around 78°C (peak top, broad endotherm at 30 to 120°C), around 191°C (peak top, extrapolated start temperature around 183°C), around 206°C (extrapolated start temperature)
Mass decrease: around 30°C to 120°C (9.7%)

### Example 2-1: crystal form I of the methanesulfonic acid salt of the compound 1

The compound 1 (2.063 g) was suspended in acetone/water (1/1) (6.2 mL). Methanesulfonic acid (0.370 g) was dissolved in acetone/water (1/1) (4.1 mL) separately, and to the suspension of the compound 1 was added the solution. The mixture was stirred for 15 minutes at 61°C. After dissolution, the solution was cooled to room temperature and then filtered under suction using a filter, and the vessel and the filter were washed with acetone/water (1/1) (1 mL) twice. To the obtained filtrate was added acetone (10.31 mL) under stirring at 64°C, and the solution was stirred for 5 minutes. To the solution was further added acetone (10.31 mL), and the mixture was stirred for 5 minutes. Heating of a water bath was stopped. To the solution was added acetone (10.31 mL) at 40°C, and the mixture was stirred for 80 minutes. To the mixture was added acetone (20.62 mL) under stirring at 40°C, and the mixture was stirred for 45 minutes at 60°C. The mixture was stirred for 16 hours at room temperature. The mixture was filtered under suction and washed with 5% aqueous acetone (4 mL) twice. The obtained solid was dried under reduced pressure for 2 hours at 40°C to give the crystal form I of the methanesulfonic acid salt of the compound 1 (2.183 g) as white powder.

The powder X-ray diffraction for the crystal form I of the methanesulfonic acid salt of the compound 1 was measured. The diffraction angles (2*θ* (°)) of major diffraction peaks and the relative intensities (Rel. Den. (%)) of the diffraction peaks are shown in table 7.

**[Table 7]**

| 2 *θ* (° ) | Rel. Den.(%) | 2 *θ* (° ) | Rel. Den.(%) |
|---|---|---|---|
| 7.0 | 34 | 17.6 | 31 |
| 10.6 | 57 | 18.6 | 17 |
| 13.7 | 19 | 20.5 | 20 |
| 14.1 | 100 | 21.6 | 15 |
| 16.2 | 42 | 24.5 | 26 |

For the identification of the crystal form I of the methanesulfonic acid salt of the compound 1, for example, a subset of the diffraction peaks selected from the group consisting of the following [2-1-1] to [2-1-7] can be used:
[2-1-1] peaks of 10.6±0.2 and 14.1±0.2;
[2-1-2] peaks of 7.0±0.2 and 10.6±0.2;
[2-1-3] peaks of 7.0±0.2, 10.6±0.2 and 14.1±0.2;
[2-1-4] peaks of 10.6±0.2, 14.1±0.2 and 16.2±0.2;
[2-1-5] peaks of 7.0±0.2, 10.6±0.2, 14.1±0.2 and 16.2±0.2;
[2-1-6] peaks of 7.0±0.2, 10.6±0.2, 14.1±0.2, 16.2±0.2 and 17.6±0.2; and
[2-1-7] peaks of 7.0±0.2, 10.6±0.2, 13.7±0.2, 14.1±0.2, 16.2±0.2, 17.6±0.2, 18.6±0.2, 20.5±0.2, 21.6±0.2, and 24.5±0.2.

Thermal analysis for the crystal form I of the methanesulfonic acid salt of the compound 1 was performed.
Endotherm: around 253°C (peak top, extrapolated start temperature around 250°C)
Mass decrease: around 27°C to 100°C (2.8%)

### Example 2-2: crystal form II of the methanesulfonic acid salt of the compound 1

The methanesulfonic acid salt of the compound 1 (200 mg) was suspended in 1,4-dioxane/water (1/1) (6.0 mL) and dissolved at 60°C. The solution was frozen in a dry ice-acetone bath, and the solid was lyophilized overnight. The lyophilized solid was dried under reduced pressure for about 7 hours at 40°C. To the solid was added MTBE (4.0 mL). The suspension was stirred for 2 hours at 53°C. The suspension was stirred overnight at room temperature. MTBE (2.0 mL) was added at room temperature, and the suspension was further stirred for 6 hours. The slurry was filtered under suction. The obtained solid was dried under reduced pressure for 2 hours at 40°C to give the crystal form II of the methanesulfonic acid salt of the compound 1 (189 mg) as white powder.

The powder X-ray diffraction for the crystal form II of the methanesulfonic acid salt of the compound 1 was measured. The diffraction angles (2*θ* (°)) of major diffraction peaks and the relative intensities (Rel. Den. (%)) of the diffraction peaks are shown in table 8.

**[Table 8]**

| 2 *θ* (° ) | Rel. Den.(%) | 2 *θ* (° ) | Rel. Den.(%) |
|---|---|---|---|
| 6.3 | 24 | 18.7 | 52 |
| 10.6 | 18 | 21.8 | 56 |
| 12.7 | 22 | 24.9 | 29 |
| 17.7 | 18 | 25.5 | 16 |
| 18.4 | 100 | | |

For the identification of the crystal form II of the methanesulfonic acid salt of the compound 1, for example, a subset of the diffraction peaks selected from the group consisting of the following [2-2-1] to [2-2-6] can be used:
[2-2-1] peaks of 21.8±0.2 and 25.5±0.2;
[2-2-2] peaks of 18.4±0.2 and 25.5±0.2;
[2-2-2] peaks of 18.4±0.2, 21.8±0.2 and 25.5±0.2;
[2-2-3] peaks of 18.4±0.2, 18.7±0.2, 21.8±0.2 and 25.5±0.2;
[2-2-4] peaks of 6.3±0.2, 12.7±0.2, 18.4±0.2, 18.7±0.2, 21.8±0.2, 24.9±0.2 and 25.5±0.2;
[2-2-5] peaks of 6.3±0.2, 10.6±0.2, 12.7±0.2, 18.4±0.2, 18.7±0.2, 21.8±0.2, 24.9±0.2 and 25.5±0.2; and
[2-2-6] peaks of 6.3±0.2, 10.6±0.2, 12.7±0.2, 17.7±0.2, 18.4±0.2, 18.7±0.2, 21.8±0.2, 24.9±0.2 and 25.5±0.2.

Thermal analysis for the crystal form II of the methanesulfonic acid salt of the compound 1 was performed.
Exotherm: around 172°C (peak top)
Endotherm: around 248°C (peak top, extrapolated start temperature around 243°C)
Mass decrease: around 27°C to 100°C (2.1%)

### Example 2-3: crystal form III of the methanesulfonic acid salt of the compound 1

The methanesulfonic acid salt of the compound 1 (300 mg) was suspended in 1,4-dioxane/water (1/1) (6.0 mL), and the suspension dissolved at 60°C. The solution was frozen in a dry ice-acetone bath, and the solid was lyophilized overnight.

To the solid (10 mg) obtained by lyophilization was added propyl acetate (0.2 mL), and the mixture was stirred for 5 days at 60°C. The slurry was filtered to give the crystal form III of the methanesulfonic acid salt of the compound 1. Using the crystal as seed crystal, the following scale up was performed using the lyophilized solid.

To the above solid (100 mg) obtained by lyophilization were added propyl acetate (5 mL) and the seed crystal (1 mg), and the mixture was stirred for 5 days at 60°C. The slurry was filtered under suction. The obtained solid was washed with propyl acetate (1 mL) and dried under reduced pressure for 5 hours at 40°C to give the crystal form III of the methanesulfonic acid salt of the compound 1 (98 mg).

The powder X-ray diffraction for the crystal form III of the methanesulfonic acid salt of the compound 1 was measured. The diffraction angles (2*θ* (°)) of major diffraction peaks and the relative intensities (Rel. Den. (%)) of the diffraction peaks are shown in table 9.

**[Table 9]**

| 2 *θ* (° ) | Rel. Den.(%) | 2 *θ* (° ) | Rel. Den.(%) |
|---|---|---|---|
| 6.4 | 24 | 18.3 | 64 |
| 12.8 | 59 | 19.3 | 38 |
| 16.8 | 25 | 22.6 | 73 |
| 17.8 | 100 | 24.3 | 47 |

For the identification of the crystal form III of the methanesulfonic acid salt of the compound 1, for example, a subset of the diffraction peaks selected from the group consisting of the following [2-3-1] to [2-3-7] can be used:
[2-3-1] peaks of 12.8±0.2 and 22.6±0.2;
[2-3-2] peaks of 17.8±0.2 and 22.6±0.2;
[2-3-3] peaks of 12.8±0.2, 17.8±0.2 and 22.6±0.2;
[2-3-4] peaks of 17.8±0.2, 18.3±0.2 and 22.6±0.2;
[2-3-5] peaks of 12.8±0.2, 17.8±0.2, 18.3±0.2 and 22.6±0.2;
[2-3-6] peaks of 6.4±0.2, 12.8±0.2, 17.8±0.2, 18.3±0.2, 19.3±0.2, 22.6±0.2 and 24.3±0.2; and
[2-3-7] peaks of 6.4±0.2, 12.8±0.2, 16.8±0.2, 17.8±0.2, 18.3±0.2, 19.3±0.2, 22.6±0.2 and 24.3±0.2.

Thermal analysis for the crystal form III of the methanesulfonic acid salt of the compound 1 was performed.
Endotherm: around 252°C (peak top, extrapolated start temperature around 248°C)
Mass decrease: around 30°C to 230°C (0.9%)

### Example 2-4: crystal form IV of the methanesulfonic acid salt of the compound 1

The methanesulfonic acid salt of the compound 1 (161 mg) was dissolved in 1,4-dioxane/water (1/1) (0.485 mL) at 50°C, and the solution was filtered. The filtrate was lyophilized. To the obtained solid was added THF/water (1/1) (0.322 mL), and the mixture dissolved at 50°C. Then, the solution was stirred at room temperature. When stirring was stopped due to the precipitated crystals, to the mixture was added THF (0.966 mL), and the suspension was stirred. THF (0.966 mL) was further added, and the suspension was stirred. The slurry was filtered under suction. The obtained solid was dried under reduced pressure for 1 hour at 40°C to give the crystal form IV of the methanesulfonic acid salt of the compound 1 (138 mg) as white powder.

The powder X-ray diffraction for the crystal form IV of the methanesulfonic acid salt of the compound 1 was measured. The diffraction angles (2*θ* (°)) of major diffraction peaks and the relative intensities (Rel. Den. (%)) of the diffraction peaks are shown in table 10.

**[Table 10]**

| 2 *θ* (° ) | Rel. Den.(%) | 2 *θ* (° ) | Rel. Den.(%) |
|---|---|---|---|
| 6.0 | 70 | 15.4 | 100 |
| 9.9 | 21 | 16.3 | 21 |
| 11.0 | 41 | 18.2 | 20 |
| 12.0 | 15 | 19.9 | 16 |
| 13.5 | 17 | 24.9 | 21 |

For the identification of the crystal form IV of the methanesulfonic acid salt of the compound 1, for example, a subset of the diffraction peaks selected from the group consisting of the following [2-4-1] to [2-4-6] can be used:
[2-4-1] peaks of 6.0±0.2 and 15.4±0.2;
[2-4-2] peaks of 6.0±0.2 and 11.0±0.2;
[2-4-3] peaks of 6.0±0.2, 11.0±0.2 and 15.4±0.2;
[2-4-4] peaks of 6.0±0.2, 9.9±0.2, 11.0±0.2 and 15.4±0.2;
[2-4-5] peaks of 6.0±0.2, 11.0±0.2, 15.4±0.2 and 16.3±0.2;
[2-4-6] peaks of 6.0±0.2, 9.9±0.2, 11.0±0.2, 15.4±0.2, 16.3±0.2, 18.2±0.2 and 24.9±0.2; and
[2-4-7] peaks of 6.0±0.2, 9.9±0.2, 11.0±0.2, 12.0±0.2, 13.5±0.2, 15.4±0.2, 16.3±0.2, 18.2±0.2, 19.9±0.2 and 24.9±0.2.

Thermal analysis for the crystal form IV of the methanesulfonic acid salt of the compound 1 was performed.
Endotherm: around 64°C (peak top, broad endotherm at 22 to 100°C), around 155°C (extrapolated start temperature)
Mass decrease: around 22°C to 100°C (8.1%)

### Example 3-1: crystal form I of the hydrochloride of the compound 1

The compound 1 (300 mg) was suspended in 1,4-dioxane/water (1/1) (7.5 mL), and to the suspension was added 1 mol/L hydrochloric acid (0.56 mL). After dissolution, the solution was lyophilized. To the obtained solid was added 2-propanol (6.0 mL), and the mixture was stirred for 30 minutes at room temperature. Then, the mixture was stirred for 2 hours at 53°C and stirred for 4 days at room temperature. The slurry was filtered under suction. The obtained solid was dried under reduced pressure for 2 hours at 43°C to give the crystal form I of the hydrochloride of the compound 1 (310 mg).

The powder X-ray diffraction for the crystal form I of the hydrochloride of the compound 1 was measured. The diffraction angles (2*θ* (°)) of major diffraction peaks and the relative intensities (Rel. Den. (%)) of the diffraction peaks are shown in table 11.

**[Table 11]**

| 2 *θ* (° ) | Rel. Den.(%) | 2 *θ* (° ) | Rel. Den.(%) |
|---|---|---|---|
| 13.1 | 100 | 19.2 | 11 |
| 13.4 | 24 | 23.2 | 14 |
| 13.7 | 14 | 23.9 | 28 |
| 14.0 | 12 | 24.6 | 12 |
| 17.4 | 16 | 26.9 | 13 |

For the identification of the crystal form I of the hydrochloride of the compound 1, for example, a subset of the diffraction peaks selected from the group consisting of the following [3-1-1] to [3-1-5] can be used:
[3-1-1] peaks of 13.1±0.2 and 17.4±0.2;
[3-1-2] peaks of 13.1±0.2, 17.4±0.2 and 23.9±0.2;
[3-1-3] peaks of 13.1±0.2, 13.4±0.2, 17.4±0.2 and 23.9±0.2;
[3-1-4] peaks of 13.1±0.2, 13.4±0.2, 17.4±0.2, 23.9±0.2 and 26.9±0.2; and
[3-1-5] peaks of 13.1±0.2, 13.4±0.2, 13.7±0.2, 14.0±0.2, 17.4±0.2, 19.2±0.2, 23.2±0.2, 23.9±0.2, 24.6±0.2 and 26.9±0.2.

Thermal analysis for the crystal form I of the hydrochloride of the compound 1 was performed.
Endotherm: around 235°C (peak top, extrapolated start temperature around 217°C)
Mass decrease: around 24°C to 200°C (3.0%)

### Example 3-2: crystal form II of the hydrochloride of the compound 1

To the compound 1 (103 mg) was added 1 mol/L hydrochloric acid (0.20 mL) at room temperature, and to the mixture were further added water (0.20 mL) and acetone (0.40 mL). The mixture was stirred for 20 minutes at 61°C. After dissolution, the solution was stirred for 1 hour at room temperature. To the solution was added acetone (1.24 mL), and the mixture was stirred for 1 hour. To the mixture was added acetone/water (20/1) (0.62 mL), and the mixture was stirred for 30 minutes. The solid in the slurry was filtered, and the solid was dried under reduced pressure for 1 hour at 43°C to give the crystal form II of the hydrochloride of the compound 1 (97 mg).

The powder X-ray diffraction for the crystal form II of the hydrochloride of the compound 1 was measured. The diffraction angles (2*θ* (°)) of major diffraction peaks and the relative intensities (Rel. Den. (%)) of the diffraction peaks are shown in table 12.

**[Table 12]**

| 2 *θ* (° ) | Rel. Den.(%) | 2 *θ* (° ) | Rel. Den.(%) |
|---|---|---|---|
| 6.2 | 78 | 13.0 | 30 |
| 8.1 | 100 | 16.4 | 25 |
| 8.4 | 31 | 25.2 | 23 |
| 10.2 | 64 | 26.4 | 38 |
| 12.1 | 55 | 27.1 | 18 |

For the identification of the crystal form II of the hydrochloride of the compound 1, for example, a subset of the diffraction peaks selected from the group consisting of the following [3-2-1] to [3-2-6] can be used:
[3-2-1] peaks of 6.2±0.2 and 8.1±0.2;
[3-2-2] peaks of 6.2±0.2, 8.1±0.2 and 10.2±0.2;
[3-2-3] peaks of 6.2±0.2, 8.1±0.2, 10.2±0.2 and 12.1±0.2;
[3-2-4] peaks of 6.2±0.2, 8.1±0.2, 10.2±0.2, 12.1±0.2 and 13.0±0.2;
[3-2-5] peaks of 6.2±0.2, 8.1±0.2, 10.2±0.2, 12.1±0.2, 13.0±0.2 and 26.4±0.2; and
[3-2-6] peaks of 6.2±0.2, 8.1±0.2, 8.4±0.2, 10.2±0.2, 12.1±0.2, 13.0±0.2, 16.4±0.2, 25.2±0.2, 26.4±0.2 and 27.1±0.2.

Thermal analysis for the crystal form II of the hydrochloride of the compound 1 was performed.
Endotherm: around 63°C (peak top, broad endotherm at 28°C to 100°C), around 189°C (peak top, extrapolated start temperature around 180°C)
Mass decrease: around 28°C to 100°C (9.7%)

### Example 3-3: crystal form III of the hydrochloride of the compound 1

To the crystal form II of the hydrochloride of the compound 1 (334 mg) obtained by the method of Example 3-2 were added acetone (1.8 mL), water (0.8 mL) and 1 mol/L hydrochloric acid (0.58 mL), and the suspension was stirred for 3 hours at 45°C. After the suspension was allowed to cool to room temperature, to the suspension was added acetone (3.3 mL), and the mixture was stirred overnight at room temperature. The slurry was filtered under suction. The obtained solid was dried under reduced pressure for 2 hours at 40°C to give the crystal form III of the hydrochloride of the compound 1 (242 mg).

The powder X-ray diffraction for the crystal form III of the hydrochloride of the compound 1 was measured. The diffraction angles (2*θ* (°)) of major diffraction peaks and the relative intensities (Rel. Den. (%)) of the diffraction peaks are shown in table 13.

**[Table 13]**

| 2 *θ* (° ) | Rel. Den.(%) | 2 *θ* (° ) | Rel. Den.(%) |
|---|---|---|---|
| 12.9 | 79 | 24.0 | 33 |
| 13.6 | 100 | 26.3 | 37 |
| 20.5 | 25 | 27.3 | 38 |
| 21.4 | 55 | 30.6 | 28 |
| 22.8 | 27 | | |

For the identification of the crystal form III of the hydrochloride of the compound 1, for example, a subset of the diffraction peaks selected from the group consisting of the following [3-3-1] to [3-3-3] can be used:
[3-3-1] peaks of 12.9±0.2 and 21.4±0.2;
[3-3-2] peaks of 12.9±0.2, 13.6±0.2 and 21.4±0.2; and
[3-3-3] peaks of 12.9±0.2, 13.6±0.2, 20.5±0.2, 21.4±0.2, 22.8±0.2, 24.0±0.2, 26.3±0.2, 27.3±0.2 and 30.6±0.2.

Thermal analysis for the crystal form III of the hydrochloride of the compound 1 was performed.
Endotherm: around 68°C (peak top, broad endotherm at 24°C to 90°C), around 191°C (peak top, broad endotherm at 150°C to 208°C), around 226°C (peak top, extrapolated start temperature around 208°C)
Mass decrease: around 28°C to 100°C (2.6%), around 100°C to 208°C (6.5%)

### Example 3-4: crystal form IV of the hydrochloride of the compound 1

The crystal form I of the methanesulfonic acid salt of the compound 1 (352 mg) obtained by the method of Example 2-1 was suspended in the 1st fluid of the dissolution test in the Japanese Pharmacopoeia (25 mL), and the suspension was shaken for 24 hours at 37°C. The suspension was left standing for 2 hours at room temperature and filtered under suction. The obtained solid was dried under reduced pressure for 3 hours at room temperature to give the crystal form IV of the hydrochloride of the compound 1 (300 mg) as white powder.

The powder X-ray diffraction for the crystal form IV of the hydrochloride of the compound 1 was measured. The diffraction angles (2*θ* (°)) of major diffraction peaks and the relative intensities (Rel. Den. (%)) of the diffraction peaks are shown in table 14.

**[Table 14]**

| 2 *θ* (° ) | Rel. Den.(%) | 2 *θ* (° ) | Rel. Den.(%) |
|---|---|---|---|
| 5.2 | 17 | 12.5 | 11 |
| 10.5 | 100 | 16.2 | 52 |
| 11.0 | 98 | 24.1 | 11 |

For the identification of the crystal form IV of the hydrochloride of the compound 1, for example, a subset of the diffraction peaks selected from the group consisting of the following [3-4-1] to [3-4-4] can be used:
[3-4-1] peaks of 10.5±0.2 and 11.0±0.2;
[3-4-2] peaks of 10.5±0.2, 11.0±0.2 and 16.2±0.2;
[3-4-3] peaks of 5.2±0.2, 10.5±0.2, 11.0±0.2 and 16.2±0.2;
[3-4-4] peaks of 5.2±0.2, 10.5±0.2, 11.0±0.2, 12.5±0.2 and 16.2±0.2; and
[3-4-5] peaks of 5.2±0.2, 10.5±0.2, 11.0±0.2, 12.5±0.2 and 16.2±0.2 and 24.1±0.2.

Thermal analysis for the crystal form IV of the hydrochloride of the compound 1 was performed.
Endotherm: around 67°C (peak top, extrapolated start temperature around 49°C), around 179°C (peak top, extrapolated start temperature around 174°C), around 216°C (peak top, extrapolated start temperature around 197°C)
Mass decrease: around 25°C to 100°C (12.9%)

### Example 4: crystal form I of the p-toluenesulfonic acid salt of the compound 1

The compound 1 (260 mg) was suspended in 1,4-dioxane/water (1/1) (6.5 mL), and to the suspension was added p-toluenesulfonic acid monohydrate (92 mg). After dissolution, the solution was frozen in a dry ice-acetone bath, and the solid was lyophilized. To the solid obtained by lyophilization was added 2-propanol (5 mL) at room temperature, and the mixture was stirred. Then, the mixture was stirred for 3 hours at 45°C and stirred for 3 days at room temperature. The slurry was filtered under suction. The obtained solid was dried under reduced pressure for 2 hours at 40°C to give the crystal form I of the p-toluenesulfonic acid salt of the compound 1 (340 mg) as white powder.

The powder X-ray diffraction for the crystal form I of the p-toluenesulfonic acid salt of the compound 1 was measured. The diffraction angles (2*θ* (°)) of major diffraction peaks and the relative intensities (Rel. Den. (%)) of the diffraction peaks are shown in table 15.

**[Table 15]**

| 2 *θ* (° ) | Rel. Den.(%) | 2 *θ* (° ) | Rel. Den.(%) |
|---|---|---|---|
| 6.1 | 95 | 16.4 | 32 |
| 12.2 | 73 | 16.7 | 71 |
| 12.9 | 32 | 20.6 | 29 |
| 13.8 | 48 | 21.2 | 30 |
| 14.0 | 64 | 24.7 | 100 |
| 15.3 | 44 | 25.4 | 62 |

For the identification of the crystal form I of the p-toluenesulfonic acid salt of the compound 1, for example, a subset of the diffraction peaks selected from the group consisting of the following [4-1] to [4-6] can be used:
[4-1] peaks of 6.1±0.2 and 24.7±0.2;
[4-2] peaks of 6.1±0.2 and 12.2±0.2;
[4-3] peaks of 6.1±0.2, 12.2±0.2 and 24.7±0.2;
[4-4] peaks of 6.1±0.2, 12.2±0.2, 14.0±0.2, 16.7±0.2 and 24.7±0.2;
[4-5] peaks of 6.1±0.2, 12.2±0.2, 14.0±0.2, 16.7±0.2, 24.7±0.2 and 25.4±0.2; and
[4-6] peaks of 6.1±0.2, 12.2±0.2, 12.9±0.2, 13.8±0.2, 14.0±0.2, 15.3±0.2, 16.4±0.2, 16.7±0.2, 20.6±0.2, 21.2±0.2, 24.7±0.2 and 25.4±0.2.

Thermal analysis for the crystal form I of the p-toluenesulfonic acid salt of the compound 1 was performed.
Endotherm: around 100°C (peak top, extrapolated start temperature around 87°C), around 236°C (peak top, extrapolated start temperature around 225°C)
Mass decrease: around 29°C to 120°C (1.9%)

### Example 5: crystal form I of the sodium salt of the compound 1

The compound 1 (50 mg) was suspended in 2-propanol/water (1/1) (0.5 mL), and to the suspension was added an aqueous solution of 1 mol/L sodium hydroxide (0.185 mL). After dissolution, to the solution was added 1 mol/L hydrochloric acid (0.093 mL), and the mixture was stirred for 3 days at room temperature. The mixture was stirred for 5 hours at 40°C and further stirred overnight at room temperature. The slurry was filtered under suction. The obtained solid was dried under reduced pressure for 3 hours at 40°C to give the crystal form I of the sodium salt of the compound 1 (34 mg) as white powder.

The powder X-ray diffraction for the crystal form I of the sodium salt of the compound 1 was measured. The diffraction angles (2*θ* (°)) of major diffraction peaks and the relative intensities (Rel. Den. (%)) of the diffraction peaks are shown in table 16.

**[Table 16]**

| 2 *θ* (° ) | Rel. Den.(%) | 2 *θ* (° ) | Rel. Den.(%) |
|---|---|---|---|
| 5.8 | 28 | 17.4 | 41 |
| 12.1 | 33 | 22.7 | 100 |
| 13.0 | 27 | 23.8 | 45 |
| 15.9 | 63 | 25.1 | 36 |
| 16.2 | 28 | 26.2 | 24 |

For the identification of the crystal form I of the sodium salt of the compound 1, for example, a subset of the diffraction peaks selected from the group consisting of the following [5-1] to [5-7] can be used:
[5-1] peaks of 5.8±0.2 and 22.7±0.2;
[5-2] peaks of 15.9±0.2 and 22.7±0.2;
[5-3] peaks of 5.8±0.2, 15.9±0.2 and 22.7±0.2;
[5-4] peaks of 5.8±0.2, 12.1±0.2, 15.9±0.2 and 22.7±0.2;
[5-5] peaks of 5.8±0.2, 12.1±0.2, 15.9±0.2, 17.4±0.2 and 22.7±0.2;
[5-6] peaks of 5.8±0.2, 12.1±0.2, 15.9±0.2, 17.4±0.2, 22.7±0.2 and 23.8±0.2; and
[5-7] peaks of 5.8±0.2, 12.1±0.2, 13.0±0.2, 15.9±0.2, 16.2±0.2, 17.4±0.2, 22.7±0.2, 23.8±0.2, 25.1±0.2 and 26.2±0.2.

Thermal analysis for the crystal form I of the sodium salt of the compound 1 was performed.
Endotherm: around 75°C (peak top, broad endotherm at 50°C to 120°C), around 197°C (peak top, extrapolated start temperature around 194°C)
Mass decrease: around 29°C to 150°C (13.9%)

### Example 6: crystal form I of the potassium salt of the compound 1

The compound 1 (100 mg) was suspended in 2-propanol/water (1/1) (1.0 mL), and to the suspension was added an aqueous solution of 1 mol/L potassium hydroxide (0.370 mL). After dissolution, to the solution was added 1 mol/L hydrochloric acid (0.185 mL) at room temperature, and the mixture was stirred for 10 minutes. To the mixture was added 2-propanol (1.25 mL) at room temperature, and the mixture was stirred for 2 hours. To the mixture was added 2-propanol (0.25 mL) at room temperature, and the mixture was stirred for 1 hour. Further, to the mixture was added 2-propanol (0.5 mL) at room temperature, and the mixture was stirred for 6 hours. The slurry was filtered and washed with 2-propanol/water (4/1) (1.0 mL) twice. The obtained solid was dried under reduced pressure for 1 hour at room temperature and for 1 hour at 40°C to give the crystal form I of the potassium salt of the compound 1 (70 mg) as white powder.

The powder X-ray diffraction for the crystal form I of the potassium salt of the compound 1 was measured. The diffraction angles (2*θ* (°)) of major diffraction peaks and the relative intensities (Rel. Den. (%)) of the diffraction peaks are shown in table 17.

**[Table 17]**

| 2 *θ* (° ) | Rel. Den.(%) | 2 *θ* (° ) | Rel. Den.(%) |
|---|---|---|---|
| 5.7 | 41 | 17.4 | 21 |
| 11.6 | 23 | 22.5 | 30 |
| 12.0 | 26 | 22.8 | 100 |
| 15.8 | 25 | 29.4 | 16 |

For the identification of the crystal form I of the potassium salt of the compound 1, for example, a subset of the diffraction peaks selected from the group consisting of the following [6-1] to [6-6] can be used:
[6-1] peaks of 5.7±0.2 and 22.8±0.2;
[6-2] peaks of 5.7±0.2, 12.0±0.2 and 22.8±0.2;
[6-3] peaks of 5.7±0.2, 11.6±0.2, 12.0±0.2 and 22.8±0.2;
[6-4] peaks of 5.7±0.2, 11.6±0.2, 12.0±0.2, 15.8±0.2, 17.4±0.2 and 22.8±0.2;
[6-5] peaks of 5.7±0.2, 11.6±0.2, 12.0±0.2, 15.8±0.2, 17.4±0.2, 22.5±0.2 and 22.8±0.2; and
[6-6] peaks of 5.7±0.2, 11.6±0.2, 12.0±0.2, 15.8±0.2, 17.4±0.2, 22.5±0.2, 22.8±0.2 and 29.4±0.2.

Thermal analysis for the crystal form I of the potassium salt of the compound 1 was performed.
Endotherm: around 70°C (peak top, broad endotherm at 23°C to 100°C), around 187°C (peak top, extrapolated start temperature around 182°C)
Mass decrease: around 23°C to 100°C (12.9%)

### Example 7: crystal form I of the calcium salt of the compound 1

The compound 1 (300 mg) was suspended in methanol/water (1/1) (3.0 mL), and to the suspension was added an aqueous solution of 1 mol/L sodium hydroxide (1.12 mL), and the mixture was stirred at room temperature. After dissolution, to the solution was added calcium chloride (62 mg) at room temperature, and the mixture was stirred for 1 hour. The mixture was stirred for 3 hours at 45°C and further stirred overnight at room temperature. The slurry was filtered under suction. The obtained solid was dried under reduced pressure (3 hours) at 43°C to give the crystal form I of the calcium salt of the compound 1 (310 mg) as white powder.

The powder X-ray diffraction for the crystal form I of the calcium salt of the compound 1 was measured. The diffraction angles (2*θ* (°)) of major diffraction peaks and the relative intensities (Rel. Den. (%)) of the diffraction peaks are shown in table 18.

**[Table 18]**

| 2 *θ* (° ) | Rel. Den.(%) | 2 *θ* (° ) | Rel. Den.(%) |
|---|---|---|---|
| 7.3 | 100 | 17.3 | 24 |
| 16.1 | 45 | 17.7 | 35 |
| 16.5 | 20 | 20.1 | 35 |
| 17.0 | 31 | 25.4 | 23 |

For the identification of the crystal form I of the calcium salt of the compound 1, for example, a subset of the diffraction peaks selected from the group consisting of the following [7-1] to [7-5] can be used:
[7-1] peaks of 7.3±0.2 and 16.1±0.2;
[7-2] peaks of 7.3±0.2, 16.1±0.2 and 20.1±0.2;
[7-3] peaks of 7.3±0.2, 16.1±0.2, 17.7±0.2 and 20.1±0.2;
[7-4] peaks of 7.3±0.2, 16.1±0.2, 17.0±0.2, 17.7±0.2 and 20.1±0.2; and
[7-5] peaks of 7.3±0.2, 16.1±0.2, 16.5±0.2, 17.0±0.2, 17.3±0.2, 17.7±0.2, 20.1±0.2 and 25.4.

Thermal analysis for the crystal form I of the calcium salt of the compound 1 was performed.
Endotherm: around 79°C (peak top, broad endotherm at 25°C to 150°C)
Mass decrease: around 25°C to 200°C (13.4%)

The chemical shift values of the ¹H-NMR spectra of the salts of Example 2-1 to Example 7 are shown in the following tables.

**[Table 19]**

| Ex. No. | Physical data |
|---|---|
| 2-1 | (DMSO-d6) *δ* ppm: 2.29 (3H, s), 2.61 (2H, brs) , 3.61 (1H, brs), 3.74-4.18 (6H, m) , 4.78 (2H, brs), 5.46 (1H, brs), 7.21 (1H, dd, J=5.2, 7.6Hz) , 7.66 (1H, dd, J=1.2, 7.6Hz), 7.70 (1H, s) , 8.07-8.12 (2H, m), 8.13 (1H, dd, J=1.2, 5.2Hz), 8.18 (1H, dd, J=2.6, 8.4Hz), 8.25-8.36 (3H, m), 8.96 (1H, dd, J=0.6, 2.4Hz), 10.83 (1H, brs), 13.12 (2H, brs) |
| 2-2 | (DMSO-d6) *δ* ppm: 2.29 (3H, s), 2.60 (2H, brs), 3.60 (1H, brs), 3.70-4.25 (6H, m), 4.76 (2H, brs), 5.45 (1H, brs), 7.20 (1H, dd, J=5.2, 7.6Hz), 7.66 (1H, dd, J=1.0, 7.6Hz) , 7.69 (1H, s), 8.06-8.12 (2H, m), 8.13 (1H, dd, J=1.2, 5.2Hz), 8.18 (1H, dd, J=2.6, 8.4Hz), 8.24-8.35 (3H, m), 8.96(1H, dd, J=0.8, 2.8Hz), 10.85(1H, brs), 13.11 (2H, brs) |
| 2-3 | (DMSO-d6) *δ* ppm : 2.30 (3H, s), 2.62 (2H, brs), 3.70 (1H, brs), 3.85-4.17 (6H, m), 4.82 (2H, brs), 5.47 (1H, brs), 7.20 (1H, dd, J=5.2, 7.6Hz) , 7.65 (1H, dd, J=1.2, 8.0Hz), 7.70 (1H, s), 8.10(2H, m), 8.13 (1H, dd, J=1.2, 5.2Hz), 818(1H, dd, J=2.4, 8.4Hz), 8.29-8.32 (3H, m), 8.96 (1H, dd, J=0.8, 2.8Hz), 10.86 (1H, brs), 13.09 (2H, brs) |
| 2-4 | (DMSO-d6) *δ* ppm: 2.29 (3H, s), 2.60 (2H, brs), 3.60 (1H, brs), 3.70-4.24 (6H, m), 4.77 (2H, brs), 5.45 (1H, brs), 7.20 (1H, dd, J=5.2, 8.0Hz), 765 (1H, d, J=7.6Hz), 7.69 (1H, s), 8.10(2H, d, J=8.8Hz), 8.13 (1H, dd, J=1.2, 5.2Hz), 8.18 (1H, dd, J=2.6, 8.4Hz), 8.25-8.34 (3H, m), 8.96(1H, d, J=2.4Hz), 10.82 (1H, brs), 13.12 (2H, brs) |

**[Table 20]**

| Ex. No. | Physical data |
|---|---|
| 3-1 | (DMSO-d6) *δ* ppm :2.60 (2H, brs), 3.62 (1H, brs), 3.74-4.20 (6H, m), 4.78(2H, brs), 5.46 (1H, brs), 7.20 (1H, dd, J=5.4, 7.6Hz), 7.65 (1H, dd, J=1.0, 7.6Hz), 7.69 (1H, s), 8.10 (2H, d, J=8.8Hz) , 8.13 (1H, dd, J=1.4, 5.2Hz), 818(1H, dd, J=2.4, 8.4Hz), 8.27-8.35 (3H, m), 8.96 (1H, d, J=2.4Hz), 11.05 (1H, brs), 13.12 (2H, brs) |
| 3-2 | (DMSO-d6) *δ* ppm :2.60 (2H, brs), 3.61 (1H, brs), 3.70-4.20 (6H, m), 4.76(2H, brs), 5.45 (1H, brs), 7.20 (1H, dd, J=5.2. 8.0Hz). 7.65 (1H, dd, J=1.2. 8.0Hz) , 7.69 (1H, s), 8.10 (2H, d, J=8.4Hz), 8.13 (1H, dd, J=1.2, 5.3Hz) , 818(1H, dd, J=2.6, 8.4Hz), 8.24-8.36 (3H, m), 8.93 (1H, d, J=2.4Hz), 11.10 (1H, brs), 13.11 (2H, brs) |
| 3-3 | (DMSO-d6) *δ* ppm: 2.56-2.70 (2H, brs), 3.56-3.79 (1H, m), 4.72-4.90 (2H, m), 5.46 (1H, brs), 7.20 (1H, dd, J=5.2, 7.6Hz), 7.65 (1H, dd, J=1.0, 8.0Hz), 7.71 (1H, s), 8.10 (2H, d, J=8.4Hz), 8.13 (1H, dd, J=1.4, 5.6Hz), 8.19 (1H, dd, J=2.4, 8.8Hz), 8.26-8.34 (3H, m), 8.96 (1H, d, J=2.4Hz), 11.32 (1H, brs), 13.12 (1H, brs) |
| 3-4 | (DMSO-d6) *δ* ppm :2.61 (2H, brs), 3.63 (1H, brs), 3.78-4.18 (6H, m), 4.80(2H, brs), 5.46 (1H, brs), 7.20 (1H, dd, J=5.4, 7.6Hz), 7.65 (1H, dd, J=1.2, 8.0Hz), 7.70 (1H, s), 8.10 (2H, d, J=8.8Hz), 8.13 (1H, dd, J=1.2 , 4.8Hz), 8.18 (1H, dd, J=2.6, 8.4Hz), 8.25-8.37(3H, m), 8.96(1H, d, J=2.4Hz), 11.16 (1H, brs), 1312 (2H, brs) |

**[Table 21]**

| Ex. No. | Physical data |
|---|---|
| 4 | (DMSO-d6) *δ* ppm: 2.29 (3H, s), 2.60 (2H, brs), 3.61 (1H, brs), 3.74-4.20 (6H, m), , 4.80 (2H, brs), 5.46 (1H, brs), 7.11 (2H, d, J=8.0Hz), 7.21 (1H, dd, J=5.0, 8.0Hz). 7.47 (2H, d, J=8.4Hz), 7.66 (1H, dd, J=1.2, 8.0Hz), 7.70 (1H, s), 8.10(2H, d, J=7.6Hz), 8.13 (1H, dd, J=1.0, 5.2Hz), 8.18 (1H, dd, J=2.4, 8.4Hz), 8.26-8.36 (3H, m), 8.96 (1H, d, J=2.4Hz), 10.84 (1H, brs), 13.12 (2H, brs) |
| 5 | (DMSO-d6) *δ* ppm: 2.16-2.30 (1H, m), 2.83-3.03 (4H, m), 3.78 (2H, s), 3.92 (3H, s), 504-516(1H, m), 7.12 (1H, dd, J=5.4, 8.0Hz), 7.32 (1H, s), 755 (1H, dd, J=1.6, 8.0Hz) , 8.06 (2H, d, J=8.4Hz) , 8.12 (1H, dd, J=1.2, 5.2Hz), 8.17 (1H, dd, J=2.6, 8.8Hz), 8.21-8.30 (3H, m), 8.93 (1H, d, J=3.2Hz) |
| 6 | (DMSO-d6) *δ* ppm: 2.16-2.29 (1H, m), 2.80-3.03 (4H, m), 3.78 (2H, s), 3.91 (3H, s), 5.04-5.15 (1H, m), 7.12 (1H, dd, J=5.2, 8.0Hz), 7.31 (1H, s), 7.55 (1H, dd, J=1.6, 8.0Hz) , 8.06 (2H, d, J=8.4Hz) , 8.12 (1H, dd, J=1.2, 5.2Hz), 8.17 (1H, dd, J=2.6, 8.8Hz), 8.20-8.30 (3H, m), 8.93 (1H, d, J=2.8Hz) |
| 7 | (DMSO-d6/D₂O = 3/2) *δ* ppm : 2.23 (1H, brs), 2.35 (1H, brs), 2.80-2.97 (3H, m) , 2.97-3.08 (1H, m), 3.68-3.88 (5H, m), 5.08 (1H, brs), 7.10-7.17 (1H, m), 7.18 (1H, s), 7.48 (1H, d, J=8.0Hz) , 7.93 (2H, d, J=8.8Hz) , 7.98 (2H, d, J=8.0Hz) , 8.03-8.17 (3H, m), 8.77 (1H, d, J=2.0Hz) |

### Test Example 4: Solid stability test

The physicochemical stability and chemical stability for the crystals of the compound 1 (crystal forms I to III), the crystals of the methanesulfonic acid salt of the compound 1 (crystal forms I, II and IV), the crystals of the hydrochloride (crystal forms I and III), the crystal form I of the p-toluenesulfonic acid salt, the crystal form I of the sodium salt and the crystal form I of the calcium salt were examined.

### (1) Method

Physicochemical stability: Each crystal was stored under open condition at 40°C. The powder X-ray diffraction of the sample was measured at the initial point and 1 month later, and the change in crystal form was confirmed. The change in properties was also observed at the same time.

Chemical stability: Each crystal was stored under open condition at 40°C. The mass of the compound 1 in the sample was measured under the following HPLC measurement conditions at the initial point and 1 month later.

### [HPLC conditions]

Detector: Ultraviolet and visible absorptiometer/ wavelength: 225 nm
Column: L-column2 ODS, 3 µm, 4.6×150 mm (Chemicals Evaluation and Research Institute)
Column temperature: Constant temperature around 40°C
Flow rate: 1.0 mL/min
Mobile phase A: Potassium dihydrogenphosphate and dipotassium hydrogenphosphate aqueous solution adjusted to 10 mmol/L with water
Mobile phase B: Acetonitrile
Mobile phase ratios:
   0 to 30 minutes: mobile phase A/mobile phase B = 88/12
   30 to 50 minutes: mobile phase A/mobile phase B = 88/12 to 25/75
   50 to 55 minutes: mobile phase A/mobile phase B = 25/75
   55 to 55.01 minutes: mobile phase A/mobile phase B = 25/75 to 88/12
   55.01 to 75 minutes: mobile phase A/mobile phase B = 88/12
Amount of injection: 5 µL
Sample cooler: 4°C
Dissolution solvent: Mixed solution (mobile phase A/acetonitrile=75/25)
Sample solution: Sample was dissolved in dissolution solvent and adjusted to about 1 mg/mL

Excluding the peaks derived from the blank, the peak areas of the compound 1 and an analogous substance were measured by automatic integration, and the mass of the compound 1 was calculated by area normalization method. Further, the amount of the mass change of the compound 1 after the storage was calculated.

### (2) Results

In the storage under open condition at 40°C, the crystals of the compound 1 and the crystals of the salts of the compound 1 were physicochemically stable with virtually no changes in crystal form and in properties. In addition, the crystals of the compound 1 and the crystals of the salts of the compound 1 were chemically stable with virtually no decrease of the amount of the compound 1 (Table 22).

Therefore, it was demonstrated that the crystal of the compound 1 or a salt thereof of the present invention has good physical properties as a drug substance.

**[Table 22]**

| Crystal Form | Amount of the mass change of the compound 1 | Crystal form change | Properties |
|---|---|---|---|
| Crystal form I of the compound 1 | No change | No change | White powder, no change |
| Crystal form II of the compound 1 | -0. 03% | No change | White powder, no change |
| Crystal form III of the compound 1 | -0. 04% | No change | White powder, no change |
| Crystal form I of the methanesulfonic acid salt of the compound 1 | -0. 01% | No change | White powder, no change |
| Crystal form II of the methanesulfonic acid salt of the compound 1 | -0. 10% | No change | White powder, no change |
| Crystal form IV of the methanesulfonic acid salt of the compound 1 | -0.27% | No change | White powder, no change |
| Crystal fonn I of the hydrochloride of the compound 1 | -0.01% | No change | White powder, no change |
| Crystal form III of the hydrochloride of the compound 1 | -0. 02% | No change | White powder, no change |
| Crystal form I of the p-toluenesulfonic acid salt of the compound 1 | - 0.02% | No change | Change from white powder to light brown powder |
| Crystal form I of the sodium salt of the compound 1 | -0.08% | No change | White powder, no change |
| Crystal form I of the potassium salt of the compound 1 | -0.02% | No change | White powder, no change |

### [Industrial Applicability]

The crystal of the compound 1 or a salt thereof of the present invention has good physical properties as a drug substance and is useful as an agent for the treatment of an inflammatory bowel disease.

## Claims

1. A crystal of the compound represented by the following formula (I): 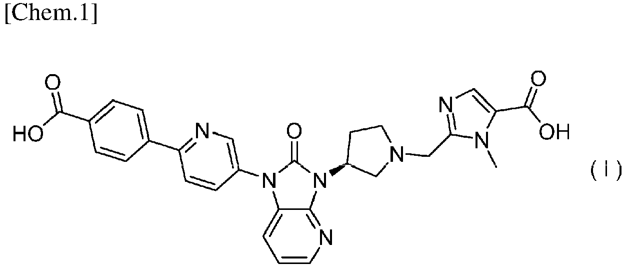 or a crystal of the salt of the compound represented by the formula (I) with an acid or a base selected from the group consisting of methanesulfonic acid, hydrogen chloride, p-toluenesulfonic acid, sodium, potassium and calcium.

2. The crystal according to claim 1 which is a crystal of the methanesulfonic acid salt having a subset of the peaks at diffraction angles (2*θ* (°)) selected from the group consisting of the following (i) to (iv) in a powder X-ray diffraction:
(i) peaks of 10.6±0.2 and 14.1±0.2;
(ii) peaks of 21.8±0.2 and 25.5±0.2;
(iii) peaks of 12.8±0.2 and 22.6±0.2; and
(iv) peaks of 6.0±0.2 and 15.4±0.2.

3. The crystal according to claim 1 which is a crystal of the methanesulfonic acid salt having a subset of the peaks at diffraction angles (2*θ* (°)) selected from the group consisting of the following (i) to (iv) in a powder X-ray diffraction:
(i) peaks of 7.0±0.2 and 10.6±0.2;
(ii) peaks of 18.4±0.2 and 25.5±0.2;
(iii) peaks of 17.8±0.2 and 22.6±0.2; and
(iv) peaks of 6.0±0.2 and 11.0±0.2.

4. The crystal according to claim 1 which is a crystal of the hydrochloride having a subset of the peaks at diffraction angles (2*θ* (°)) selected from the group consisting of the following (i) to (iv) in a powder X-ray diffraction:
(i) peaks of 13.1±0.2 and 17.4±0.2;
(ii) peaks of 6.2±0.2 and 8.1±0.2;
(iii) peaks of 12.9±0.2 and 21.4±0.2; and
(iv) 10.5±0.2 and 11.0±0.2.

5. The crystal according to claim 1 which is a crystal of a salt having a subset of the peaks at diffraction angles (2*θ* (°)) selected from the group consisting of the following (i) to (iv) in a powder X-ray diffraction:
(i) a p-toluenesulfonic acid salt, having peaks of 6.1±0.2 and 24.7±0.2;
(ii) a sodium salt, having peaks of 5.8±0.2 and 22.7±0.2;
(iii) a potassium salt, having peaks of 5.7±0.2 and 22.8±0.2; and
(iv) a calcium salt, having peaks of 7.3±0.2 and 16.1±0.2.

6. The crystal according to claim 1 which is a crystal of the compound having a subset of the peaks at diffraction angles (2*θ* (°)) selected from the group consisting of the following (i) to (iii) in a powder X-ray diffraction:
(i) peaks of 10.1±0.2 and 18.0±0.2;
(ii) peaks of 12.5±0.2 and 17.0±0.2; and
(iii) peaks of 11.1±0.2 and 14.1±0.2.

7. The crystal according to claim 1 which is a crystal of the compound having a subset of the peaks at diffraction angles (2*θ* (°)) selected from the group consisting of the following (i) to (iii) in a powder X-ray diffraction:
(i) peaks of 7.6±0.2 and 18.0±0.2;
(ii) peaks of 11.4±0.2 and 17.0±0.2; and
(iii) peaks of 10.5±0.2 and 11.1±0.2.

8. The crystal according to claim 1 which is a crystal of the compound having the peaks at diffraction angles (2*θ* (°)) of 7.6±0.2, 9.0±0.2, 10.1±0.2, 13.0±0.2, 17.1±0.2, 18.0±0.2, 19.1±0.2, 21.1±0.2, 21.7±0.2, 23.4±0.2, 26.2±0.2 and 27.5±0.2 in a powder X-ray diffraction.

9. A pharmaceutical composition comprising the crystal according to any one of claims 1 to 8 and a pharmaceutical additive.

10. The pharmaceutical composition according to claim 9 which is a pharmaceutical composition for use in the treatment of an inflammatory bowel disease.

11. The pharmaceutical composition according to claim 10, wherein the inflammatory bowel disease is ulcerative colitis or Crohn's disease.

12. A crystal of the compound represented by the following formula (I): 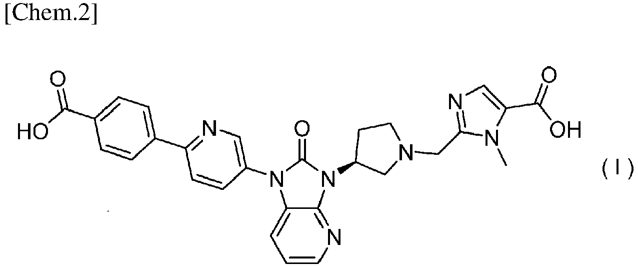 or a salt thereof.
